# EUROPEAN PATENT SPECIFICATION

(11) **EP 2 261 660 B1**
(45) Date of publication and mention of the grant of the patent: **07.08.2013**
(21) Application number: 09715199.7
(22) Date of filing: 26.02.2009
(51) Int. Cl.: G01N 33/53

(54) **BIOMARKER FOR THE ESTIMATION OF ACUTE RENAL DISORDER AND PROGNOSIS OF THE DISORDER, AND USE OF THE BIOMARKER**
BIOMARKER ZUR ABSCHÄTZUNG VON AKUTER NIERENKRANKHEIT UND PROGNOSE DER KRANKHEIT SOWIE VERWENDUNG DES BIOMARKERS
BIOMARQUEUR POUR L'ESTIMATION D'UN TROUBLE RÉNAL AIGU ET LE PRONOSTIC DU TROUBLE, ET UTILISATION DU BIOMARQUEUR

(30) Priority: 29.02.2008 JP 2008048954; 27.11.2008 JP 2008302443; 27.11.2008 JP 2008302444
(43) Date of publication of application: 15.12.2010
(73) Proprietor: National University Corporation Nagoya University, Aichi 464-8601 (JP)
(72) Inventor: KADOMATSU, Kenji, Nagoya-shi Aichi 464-8601 (JP); YUZAWA, Yukio, Nagoya-shi Aichi 464-8601 (JP); HAYASHI, Hiroki, Nagoya-shi Aichi 464-8601 (JP); MATSUO, Seiichi, Nagoya-shi Aichi 464-8601 (JP); IKEMATSU, Shinya, Nago-shi Okinawa 905-2192 (JP)
(74) Representative: Müller Fottner Steinecke
(86) International application number: PCT/JP2009/000863
(87) International publication number: WO 2009/107384

(56) References cited:
- JP-A- 2000 266 750
- US-A1- 2005 214 292
- SHINYA IKEMATSU ET AL: "High levels of urinary midkine in various cancer patients", BIOCHEMICAL AND BIOPHYSICAL RESEARCH COMMUNICATIONS, ACADEMIC PRESS, SAN DIEGO, CA; US, vol. 306, 1 January 2003 (2003-01-01), pages 329-332, XP007917031, ISSN: 0006-291X, DOI: DOI:10.1016/S0006-291X(03)00984-7
- DEVARAJAN P: "Emerging urinary biomarkers in the diagnosis of acute kidney injury", EXPERT OPINION ON MEDICAL DIAGNOSTICS, INFORMA HEALTHCARE, GB, vol. 2, no. 4, 1 January 2008 (2008-01-01) , pages 387-398, XP009121130, ISSN: 1753-0059, DOI: DOI:10.1517/17530059.2.4.387
- KAWAI HANAYO ET AL: "Lack of the growth factor midkine enhances survival against cisplatin-induced renal damage", AMERICAN JOURNAL OF PATHOLOGY, AMERICAN SOCIETY FOR INVESTIGATIVE PATHOLOGY, US, vol. 165, no. 5, 1 November 2004 (2004-11-01), pages 1603-1612, XP009144156, ISSN: 0002-9440
- SEIICHI MATSUO: 'Jin Shikkan Chiryo no Atarashii Tenkai' JOURNAL OF JAPANESE SOCIETY FOR DIALYSIS THERAPY vol. 18, no. 2, 2003, pages 187 - 191
- WAICHI SATO ET AL.: 'Midkine is involved in neutrophil infiltration into the tubulointerstitium in ischemic renal injury' J IMMUNOL vol. 167, no. 6, 2001, pages 3463 - 3469
- YASUO ADACHI ET AL.: 'A Malignant Rhabdoid Tumor of the Kidney Occurring Concurrently with a Brain Tumor.' REPORT OF A CASE, SURG TODAY vol. 30, no. 3, 2000, pages 298 - 301
- TOMOKI KOSUGI ET AL.: 'Tonyobyosei Jinsho Shino ni Okeru Seicho Inshi Midkine no Kan'yo' TONYOBYO GAPPEISHO vol. 20, no. SUPPLE, 2006, page 73
- SHINYA IKEMATSU ET AL.: 'High levels of urinary midkine in various cancer patients' BIOCHEM BIOPHYS RES COMMUN vol. 306, no. 2, 2003, pages 329 - 332
- YUKIO YUZAWA: 'Kyusei Jinshogai (AKI) no Aratana Biomarker' SEIBUTSU SHIRYO BUNSEKI vol. 32, no. 1, 25 January 2009, page 30
- HIROKI HAYASHI ET AL.: 'Nyochu midkine wa Eibin na Kyusei Nyosaikan Shogai Marker de aru' THE JAPANESE JOURNAL OF NEPHROLOGY vol. 50, no. 3, 25 April 2008, page 258

## Description

### TECHNICAL FIELD

The present invention relates to a novel biomarker that is useful for examination of acute kidney injury as well as use of the biomarker.

### [BACKGROUND ART]

Diagnoses of acute kidney injury (hereinafter, also referred to as "AKI") are carried out by using urine output, a serum creatinine (Cr) value, serum urea nitrogen, urinary NAG, β2-microglobulin value, and the like, as indicators. However, any indicator does not have diagnosis sensitivity such that pathologic conditions in an acute stage can be diagnosed sharply. Actually, diagnosis is carried out comprehensively based on the examination findings and clinical findings. Therefore, it takes a long time to start effective treatment, thus deteriorating the treatment results.

In spite of rapid progress of diagnosis and treatment, the mortality rate of patients with serious acute renal failure in intensive care units (ICU) has not been remarkably changed at around 50% in the past 50 years, posing a problem to be solved. Diseases presenting AKI include, for example, postoperative conditions of open heart surgery or aorta replacement surgery, septicemia, conditions after organ transplantation, and the like. Many of these diseases require postoperative ICU management, and require understanding of the pathologic conditions on an hourly basis after the onset of such diseases. Actually, without early diagnosis of AKI and early treatment interventions, life prognosis would not be improved. In order to cope with this, recently, it has been particularly emphasized that it is necessary to regard pathologic conditions in which the renal function is rapidly deteriorated as AKI, to classify the AKI into stages, and to start diagnosis and treatment interventions in the earlier stage.

In the present situation, diagnosis of AKI is generally carried out using the increase of serum Cr as an indicator. However, since it takes two to three days for serum Cr to increase after the actual onset of renal disorder, at the time of diagnosis of AKI, timing of treatment intervention may have already been missed in most cases. Therefore, serum Cr is insufficient as a diagnostic marker for AKI. Thus, it is urgent to establish an early biomarker.

For treatment of AKI, clinical examinations of novel therapeutic agents such as anti-apoptosis/anti-necrosis agents, anti-inflammatory agents, anti-septic agents, various growth factors, and vasodilator drugs have been carried out to date, but satisfactory results have not been obtained. This is because backgrounds of patients are complicated and causes of AKI are various. Furthermore, this is in particular because lack of early biomarker suitable for diagnosis of AKI makes it impossible to carry out early intervention.

Recently, as a novel urinary biomarker for AKI, it has been reported that neutrophil gelatinase-associated lipocaline (NGAL) (Non-patent Documents 1 and 7), interleukin 18 (IL-18) (Non-patent Documents 2 and 7), kidney injury molecule-1 (KIM-1) (Non-patent Documents 3 and 7), liver type fatty acid-binding protein (L-FABP) (Non-patent Documents 4, 5 and 7), and the like, are useful (Non-patent Document 6). In addition, Patent Document 1 discloses midkine (MDK) as a novel therapeutic agent for lupus nephritis (LN), an inflammation of the kidney caused by systemic lupus erythematosus (SLE), an autoimmune rheumatic disease, commonly known as lupus.
[Non-patent Document 1] Mishra J, et al., Lancet. 2005 Apr 2-8; 365 (9466): 1231-8.
[Non-patent Document 2] Prikh CR et al., Kidney Int. 2006 Jul; 70(1):199-203. Epub 2006 May 17.
[Non-patent Document 3] Han WK et al., Kidney Int. 2002 Jul; 62 (1): 237-44.
[Non-patent Document 4] Nakamura T. et al., American journal of Kidney Diseases, Vol. 47, No. 3(March), 2006: pp439-444
[Non-patent Document 5] Yamamoto T. et al., J Am Soc Nephrol 18: 2894-2902, 2007
[Non-patent Document 6] Coca SG. et al., Kidney Int 19: Dec Review, 2007
(Non-patent Document 71 Devaraian P., Expert Opin. Med. Diagn. 2008 2(4): 387-398.
[Patent Document 1] United States Patent Application Publication No. US 2005/0214292 A1.

### [DISCLOSURE OF THE INVENTION]

### [Problems to be Solved by the Invention]

Under the above-mentioned circumstances, an object of the present invention is to provide a biomarker for early grasping the possibility of the onset of AKI. Furthermore, the present invention also has an object to provide use of the biomarker, for example, an examination method of AKI. Furthermore, the present invention also has an object to provide a biomarker useful for understanding prognosis associated with a renal function and use thereof. In addition, the present invention also has an object to provide a biomarker useful for differential diagnosis of AKI. Furthermore, for examplatorv purposes only, provision of a biomarker useful for understanding prognosis associated with a renal function and use thereof, a biomarker useful for differential diagnosis of AKI, as well as use of the biomarker, for example, a differentiation method of AKI was studied herewith.

### [Means to Solve the problem]

From the viewpoint of the above-mentioned objects, the present inventors have paid attention to midkine (referred to as "MK"), and studied its possibility as a biomarker. Firstly, the present inventors have examined the urinary MK concentration in various kidney diseases. As a result, they have found the increase of the urinary midkine concentration in patients with acute tubular necrosis (ATN) that is AKI in a narrow sense. On the other hand, the present inventors have examined the relation between AKI after abdominal aortic aneurysm surgery and the urinary MK concentration. As a result, they have found that cases in which the urinary MK concentration is increased during the surgery developed AKI after the surgery. That is to say, surprising findings have been obtained in which the urinary MK concentration is increased in the extremely early stage in patients with the future onset of the AKI. In this way, it has been shown that the AKI onset can be predicted from the extremely early stage, that is, during surgery. Furthermore, the present inventors have examined the relation between a renal function of the transplanted kidney after kidney transplantation and the urinary MK concentration; the relation between the kidney disorder due to contrast media and the urinary MK concentration; the relation between cases undergoing partial nephrectomy and the urinary MK concentration, and further, the urinary MK concentration in minimal change cases. As a result, it is shown that the urinary MK concentration is extremely effective as an indicator for early prediction of the onset of AKI. In other AKI cases, the same results are shown. Thus, as a result of intensive study by the present inventors, it is shown that urinary MK is a useful indicator (biomarker) for understanding the possibility of the onset of AKI.

On the other hand, the examination of the relation between cases undergoing partial nephrectomy and the urinary MK concentration has proved that the urinary MK concentration is useful as an indicator for estimation of prognosis. Furthermore, as a result of the examination of the relation between acute kidney injury progressing after chemotherapy or in nephrotic syndrome and the urinary MK concentration, it has been revealed that the urinary MK concentration is useful as an indicator for estimation of prognosis of not only cases undergoing partial nephrectomy but also wide variety of cases.

On the other hand, it was proved that the urinary MK concentration is remarkably high in patients that have been diagnosed to have acute tubular necrosis (ATN) by pathological diagnosis. Moreover, from the results of analysis of the receiver operating characteristic curve (ROC curve), extremely high sensitivity and specificity are shown. Furthermore, as a result of comparative examination with existing biomarkers such as β-N-acetyl-D-glucosaminidase (NAG), urinary neutrophil gelatinase-associated lipocalin (NGAL), and urinary interleukin 18 (IL-18), AUC (area under the ROC curve) of urinary MK is significantly higher than that of the existing biomarkers, thus proving that urinary MK is the most excellent as a biomarker for differentiation of AKI. As mentioned above, as a result of the intensive study by the present inventors, findings that urinary MK is extremely excellent as a biomarker for differentiation of AKI have been obtained.

The present application provides the below-mentioned inventions made mainly based on the above-mentioned findings. A method, a biomarker, a kit and a reagent for estimating-prognosis and for differentiation of acute kidney injury are disclosed as examplatory embodiments.
[1] A biomarker for early detection of acute kidney injury, including midkine.
[2] A method for examining acute kidney injury, wherein an amount of urinary midkine is used as an indicator.
[3] The examination method described in [2], which includes the following steps (1) and (2):
   (1) detecting urinary midkine; and
   (2) determining a possibility of the onset of acute kidney injury based on a detection result.
[4] The examination method described in [2] or [3], wherein the acute kidney injury is acute tubular necrosis.
[5] The examination method described in [2] or [3], wherein the acute kidney injury is ischemic acute kidney injury, toxic acute kidney injury, or septic acute kidney injury.
[6] The examination method described in [5], wherein the ischemic acute kidney injury is postoperative ischemic acute kidney injury.
[7] The examination method described in [6], wherein the step (1) is carried out by using urine collected from a time of a surgery that causes an ischemic state to one day after the surgery.
[8] The examination method described in [6], wherein the step (1) is carried out by using urine collected in three hours from a start of the surgery that causes an ischemic state.
[9] The examination method described in any one of [6] to [8], wherein the surgery is large blood vessel surgery, open heart surgery using an artificial heart lung machine, organ transplantation, or partial nephrectomy for a renal tumor.
[10] The examination method described in any one of [3] to [9], further including the following step (1') in addition to the step (1), wherein the step (2) determines the possibility of the onset of acute kidney injury based on the detection results of the step (1) and step (1'):
   (1') detecting one or more biomarkers selected from the group consisting of urinary neutrophil gelatinase-associated lipocalin (NGAL), urinary interleukin 18 (IL-18), urinary kidney disorder molecule-1 (KIM-1), β-N-acetyl-D-glucosaminidase (NAG), and urinary liver type fatty acid-binding protein (L-FABP).
[11] The examination method described in [3], wherein in the step (1) urinary midkine is detected by using urine of patients with minimal change.
[12] A reagent for examining acute kidney injury, including an anti-midkine antibody.
[13] A kit for examination of acute kidney injury, including a reagent described in [12].
[14] A biomarker for estimation of prognosis associated with a renal function, including midkine.
[15] A method for estimating prognosis associated with a renal function, wherein an amount of urinary midkine is used as an indicator.
[16] The method for estimating prognosis described in [15], wherein prognosis of ischemic acute kidney injury, toxic acute kidney injury, or septic acute kidney injury is estimated.
[17] The method for estimating prognosis described in [15], wherein the method comprises detecting urinary midkine after kidney transplantation or partial nephrectomy, and estimating prognosis according to the following criterion,
   the increase of an amount of urinary midkine observed from six hours after the surgery to 48 hours after the surgery is transitional, the prognosis is excellent, and the increase of the amount of urinary midkine observed from six hours after the surgery to 48 hours after the surgery is not transitional and the increased value is protracted or maintained, the prognosis is poor.
[18] The method for estimating prognosis described in [17], wherein urinary midkine is detected by using urine as a specimen, which is derived from the affected kidney and/or urine derived from the healthy kidney.
[19] A reagent for estimation of prognosis associated with a renal function, including an anti-midkine antibody.
[20] A kit for estimation of prognosis associated with a renal function, including a reagent described in [19].
[21] A biomarker for differentiation of acute kidney injury, including midkine.
[22] A differentiation method of acute kidney injury, wherein an amount of urinary midkine is used as an indicator.
[23] A differentiation method described in [22], including the steps (1) and (2):
   (1) detecting urinary midkine; and
   (2) determining whether or not a cause of the acute kidney injury is acute tubular disorder based on the detection result.
[24] The differentiation method described in [22] or [23], wherein the acute kidney injury is acute tubular necrosis.
[25] The differentiation method described in [22] or [23], wherein the acute kidney injury is ischemic acute kidney injury.
[26] The differentiation method described in [25], wherein the ischemic acute kidney injury is postoperative ischemic acute kidney injury.
[27] The differentiation method described in any one of [23] to [26], further including the following step (1') in addition to step (1), wherein the step (2) determines whether or not a cause of the acute kidney injury is acute tubular disorder based on the detection results of the steps (1) and step (1'):
   (1') detecting one or more biomarkers selected from the group consisting of urinary neutrophil gelatinase-associated lipocalin (NGAL), urinary interleukin 18 (IL-18), urinary kidney disorder molecule-1 (KIM-1), β-N-acetyl-D-glucosaminidase (NAG), and urinary liver type fatty acid-binding protein (L-FABP).
[28] A reagent for differentiation of acute kidney injury, including an anti-midkine antibody.
[29] A kit for differentiation of acute kidney injury, including a reagent described in [28].

### [BRIEF DESCRIPTION OF THE DRAWINGS]

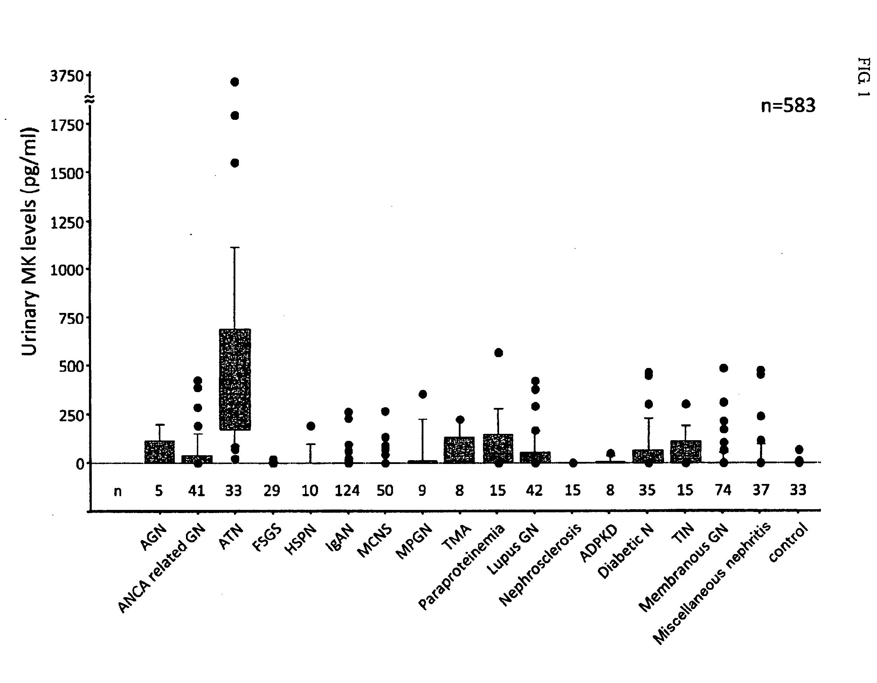

Fig. 1 is a graph showing the urinary MK concentrations of patients with kidney disease. The abscissa shows, for the left side, patients with acute glomerular nephritis (AGN) (five patients), patients with ANCA-related glomerular nephritis (anti-neutrophil cytoplasmic antibody related GN) (41 patients), patients with acute tubular necrosis (ATN) (33 patients), patients with focal segmental glomerulosclerosis (FSGS) (29 patients), patients with Henoch-Schönlein purpura nephritis (HSPN) (10 patients), patients with IgA nephropathy (IgAN) (124 patients), patients with minimal change nephrotic syndrome (MCNS) (50 patients), patients with membranoproliferative glomerulonephritis (MPGN) (nine patients), patients with thrombotic microangiopathy (TMA) (eight patients), patients with paraproteinemia-related nephropathy (15 patients), patients with lupus nephritis (Lupus GN) (42 patients), patients with nephrosclerosis (15 patients), patients with autosomal dominant polycystic kidney disease (ADPKD) (8 patients), patients with diabetic nephropathy (Diabetic N) (35 patients), patients with tubulointerstitial nephritis (TIN) (15 patients), patients with membranous nephropathy (membranous GN) (74 patients), patients with miscellaneous nephritis (37 patients), and healthy volunteers (controls) (33 patients); and the ordinate shows the urinary MK concentration (pg/ml).
Fig. 2 is a graph showing an analysis result of the ROC curve (receiver-operating characteristic curve) of the urinary MK concentration. The abscissa shows (1 - specificity), and the ordinate shows sensitivity. An area under the ROC curve is 0.965. When the cut-off value is set to be 70 pg/ml, the diagnosis sensitivity and the specificity are 97.0% and 90.3%, respectively.
Fig. 3 is graphs showing comparisons between the urinary MK and the existing urinary biomarkers (NAG, NGAL, and IL-18), respectively. Total 583 cases are divided into three groups, that is, patients with ATN (acute tubular necrosis), controls (healthy subjects), and patients with miscellaneous kidney disease. The concentration of each urinary biomarker is shown in box-and-whisker plot.
Fig. 4 shows comparison of the ROC curves for various urinary biomarkers. By using an actual measurement value (value that is not normalized by the urinary Cr concentration) of each urinary biomarker, the ATN group and the other groups (excluding healthy subjects) are differentiated. AUC shows the highest value in urinary MK.
Fig. 5 shows comparison of the ROC curves for various urinary biomarkers. By using a value normalized by the urinary Cr concentration, the ATN group and the other groups (excluding healthy subjects) are differentiated. Similar to the case using the actual measurement value, AUC shows the highest value in the urinary MK.
Fig. 6 shows comparison of AUC for various urinary biomarkers. Comparison between the AUC of urinary MK and the AUC of existing urinary biomarkers is carried out. Upper part shows a comparison using actual measurement values, and lower part shows a comparison using values normalized by urinary Cr. It is shown that in both the case using actual measurement values and the case using a value normalized by urinary Cr, urinary MK is more excellent than any other biomarkers. Note here that SE denotes standard error.
Fig. 7 shows comparison of the sensitivity and specificity when a plurality of biomarkers are combined. In the combination expressed by a mark "+," when all the biomarkers included in the combination show positive (not less than cut-off value), the result is determined to be positive. Combination expressed by a mark "/," when at least one of the biomarkers contained in the combination shows positive (not less than cut-off value), the result is determined to be positive.
Fig. 8 shows backgrounds of patients undergoing an abdominal aortic aneurysm replacement surgery.
Fig. 9 shows findings of the preoperative test of the patients undergoing an abdominal aortic aneurysm replacement surgery.
Fig. 10 shows the process of the abdominal aortic aneurysm replacement surgery and times at which the urinary MK concentration is measured. Urine was collected over time (at the time points shown by arrows), the urinary MK concentration was measured by ELISA.
Fig. 11 is a graph showing the change (ΔCr) of the urinary MK concentration (U-MK) and the amount of urinary creatinine (Cr). ●: urinary MK concentration of the AKI group, and ○: urinary MK concentration of the non-AKI group. ◆ : change amount (%) of urinary Cr of the AKI group, and 0: change amount (%) of urinary Cr of the non-AKI group.
Fig. 12 is a graph showing the change of the urinary β2 microglobulin concentration. β2 microglobulin did not allow prediction of acute kidney injury (AKI) during surgery that would be onset after surgery.
Fig. 13 is a graph showing the change of urinary NAG concentration. NAG did not allow prediction of acute kidney injury (AKI) during surgery that would be onset after surgery.
Fig. 14 is a graph showing the change of urinary cystatin C concentration. Urinary cystatin C did not allow prediction of acute kidney injury (AKI) during surgery that would be onset after surgery.
Fig. 15 shows graphs showing the change of concentrations of various biomarkers. The change of concentration of urinary MK (U-MK) is compared with that of existing biomarkers. The urinary MK concentration (U-MK) increases in an especially earlier stage as compared with the urinary IL-18 concentration (U-IL18) and the urinary NGAL concentration (U-NGAL).
Fig. 16 is a table showing the transition of the urinary midkine (MK) concentration in a case undergoing cardiac valve replacement surgery using an artificial heart lung machine.
Fig. 17 shows a protocol of an experiment (relation between a renal function of a transplanted kidney in kidney transplantation and the urinary midkine (MK) concentration).
Fig. 18 is a table showing measurement results of the serum creatinine concentration and the urinary midkine (MK) concentration immediately after kidney transplantation. The upper table shows the transition of the amount of serum creatinine and the MK concentration of the initial urine. The lower table shows the transition of the urinary MK concentration after transplantation. PGF: prompt graft function, DGF: delayed graft function.
Fig. 19 shows an outline of an experiment protocol (relation between kidney disorders by contrast media and the urinary midkine (MK) concentration).
Fig. 20 shows an outline of an experiment protocol (relation between a case undergoing partial nephrectomy for superficial early kidney cancer and the urinary midkine (MK) concentration).
Fig. 21 shows an outline of an experiment protocol (relation between a case undergoing partial nephrectomy for superficial early kidney cancer and the urinary midkine (MK) concentration).
Fig. 22 is graphs showing the change of the MK concentration of urine derived from the affected kidney (urine from the renal pelvis) (left graph) and the change of the MK concentration of urine from the urinary bladder in a non-AKI patient (right graph).
Fig. 23 is graphs showing the change of the MK concentration of urine derived from the affected kidney (urine from the renal pelvis) (left graph) and the change of the MK concentration of urine from the urinary bladder in an AKI patient (right graph).
Fig. 24 is graphs showing the change of the MK concentration of urine derived from the affected kidney (urine from the renal pelvis) (left graph) and the change of the MK concentration of urine from the urinary bladder in an AKI patient (right graph).
Fig. 25 is a table showing the change of the urinary MK concentration in serious cases.
Fig. 26 is a table showing change of the urinary MK concentration of patients with kidney disorder after chemotherapy using MTX.
Fig. 27 shows comparison of the urinary MK concentration between patients with minimal change nephrotic syndrome (MCNS) with complication of acute tubular necrosis (ATN) and patients with minimal change nephrotic syndrome (MCNS) without complication.
Fig. 28 is a graph showing the relation between the urinary MK concentration and the plasma MK concentration. For an ATN group and a group of kidney diseases other than ATN (515 cases), the urinary MK concentration and the plasma MK concentration were measured and plotted. Pearson product-moment correlation coefficient is 0.23 (95% confidence interval is 0.15 to 0.31, p < 0.001).
Fig. 29 is a graph showing the relation between the urinary MK concentration and the plasma MK concentration. For patients undergoing an abdominal aortic aneurysm replacement surgery, the urinary MK concentration and the plasma MK concentration were measured and plotted.
Fig. 30 shows the principle of MK affinity chromatography (left) and the results (right). A band that is predicted to be a MK binding protein is denoted by X. X' is predicted to be a fragment of X.
Fig. 31 is a table showing a comparison of the urinary MK concentration and the plasma MK concentration when heparin is administered to patients with amyloid kidney-complicated chronic rheumatoid arthritis. When heparin is administered, the plasma MK concentration is increased, but the urinary MK concentration is not increased.
Fig. 32 present that there is no relation between the increase in the plasma MK concentration and the urinary MK concentration. In case 1 (control), cases 2 to 5 presenting nephrotic syndrome with different selectivity, plasma MK concentration is high but the urinary MK concentration is under sensitivity. On the contrary, in ATN of cases 6-9, regardless of the plasma MK concentration, the urinary MK concentration is high.

### [BEST MODE OF CARRYING OUT THE INVENTION]

### 1. Early Detection of Acute Kidney Injury (AKI)

A first aspect of the present invention relates to early detection of acute kidney injury. In this aspect, a biomarker for the early detection of acute kidney injury and use thereof are provided.

### 1-1. Biomarker for Early Detection of Acute Kidney Injury

The "biomarker for early detection of acute kidney injury (hereinafter, also referred to as "AKI") in this specification denotes a biomolecule used as an indicator for a possibility of the onset of AKI. The "AKI" is a general name of pathologic conditions in which a renal function is rapidly deteriorated. Therefore, the "AKI includes prerenal kidney injury, renal kidney injury (acute tubular necrosis (ATN), tubulointerstitial nephritis, glomerular nephritis, and the like), and postrenal kidney injury. Glomerular disease and urinary tract infectious disease are not included in the "AKI" when they do not cause acute tubular disorder secondarily. Therefore, the present invention can be also applied to glomerular diseases and urinary tract infectious diseases when they cause acute tubular disorder secondarily.

The AKI in a narrow sense denotes prerenal kidney injury, that is, ATN that is a pathologic condition primarily causing acute tubular disorder. The present invention is particularly effective for early detection of AKI in a narrow sense. The ATN includes ischemic AKI, toxic AKI, or septic AKI. Examples of the ischemic AKI include AKI developed after surgeries, for example, a surgery of a large blood vessel such as an abdominal aortic aneurysm replacement surgery, open heart surgery such as cardiac valve replacement surgery using an artificial heart lung machine, organ transplantation, or partial nephrectomy of a renal tumor; AKI developed accompanying congestive heart failure, administration of contrast media (for example, examination using contrast media for angina pectoris, myocardial infarct, and the like), hepatorenal syndrome or thrombotic microangiopathy; AKI caused by drugs such as calcineurin inhibitor, angiotensin converting enzyme inhibitor, angiotensin II receptor antagonist,non-steroidal anti-inflammatory analgesic, and the like. However, the AKI is not necessarily limited to these examples. On the other hand, examples of the toxic AKI include AKI caused by exogenous factors such as drugs, for example, an antibacterial agent and an anticancer drug, and endogenous factors such as heme pigment (hemoglobinuria, myoglobinuria), abnormal protein in, for example, multiple myeloma, crystalline components such as uric acid and methotrexate, and the like.

The biomarker of the present invention consists of midkine (MK). MK is a proliferation differentiation factor that has been found as a product of a retinoic acid response gene, and is composed of basic amino acid and cysteine-rich polypeptide having a molecular weight of 13 kDa (Kadomatsu, K. et al.: Biochem. Biophys. Res. Commun., 151: 1312-1318; Tomomura, M. et al.: J. Biol. Chem., 265: 10765-10770, 1990). MK has multiple activities such as cell proliferation, chemotaxis, angiogenesis, induction of inflammation, fibrinolytic activity, and the like. Furthermore, it is suggested that the serum MK level is a promising tumor marker (Ikematsu. S et al., Br. J. Cancer. 2000; 83: 701-706).

An amino acid sequence of MK registered in public database (GenBank, ACCESSION:NP _001012333, DEFINITION: midkine [Homo sapiens]) is shown in SEQ ID NO 1 of the sequence listing. Note here that not MK existing in a living body but MK in a specimen collected from a living body (that is to say, MK separated from a living body) is used as the biomarker of the present invention.

### 1-2. Examination Method of Acute Kidney Injury (AKI)

The present invention also provides an examination method of AKI as one of uses of the biomarker of the present invention. The examination method of the present invention is used for determination of the possibility of the onset of AKI (early prediction of the onset). Preferably, it is used for determination of the possibility of the onset of AKI in a narrow sense, that is, the possibility of the onset of acute tubular necrosis (ATN). The examination method of the present invention is useful for determination of the possibility of the onset of ischemic AKI, toxic AKI, and septic AKI.

The examination method of the present invention may be carried out at the time of hospital admission or when a patient enters intensive care unit (ICU). The examination method of the present invention may be carried out in order to determine whether or not minimal change progresses to AKI. Note here that in "minimal change" that is the most frequent cause of nephrotic syndrome, it is known that 10 -20% of the patients presents oliguria acute renal failure.

In the examination method of the present invention, an amount of the urinary MK is used as an indicator for determination. Specifically, in the examination method of the present invention, the following steps (1) and (2) are carried out.
(1) detecting urinary MK; and
(2) determining a possibility of the onset of AKI based on the detection result.

### Step (1)

In step (1), a urine specimen collected from a subject is prepared and MK (urinary MK) existing therein is detected. The "detecting urinary MK" in this specification has the same meaning as measuring an amount of the urinary MK. Strict measurement is not essential, the amount of urinary MK may be measured by semi-quantitative measurement. For example, the detection is carried out such that determination of whether or not the amount of urinary MK exceeds a predetermined reference amount can be made.

The detection method of the urinary MK is not particularly limited. However, immunologic technique is preferably used. The immunologic technique enables the urinary MK concentration to be measured rapidly and with high sensitivity. Furthermore, the immunologic technique can be carried out in an easy and simple manner. In the measurement of the urinary MK concentration by the immunologic technique, a substance having a specific binding activity with respect to MK is used. As the substance, an anti-MK antibody is generally used. However, the substance is not necessarily limited to the anti-MK antibody, and any substances can be used as long as they have a specific binding activity with respect to MK and capable of measuring the binding amount.

Examples of the measurement method include a latex agglutination method, a fluorescence immunoassay (FIA) method, an enzyme immunoassay (EIA) method, a radioimmunoassay (RIA) method, and a Western blotting method. Preferable measurement method can be a FIA method and an EIA method (including an ELISA method). With these methods, detection can be carried out with high sensitivity, rapidly and in a simple and easy manner. In the FIA method, a fluorescent labeled antibody is used, and an antigen-antibody complex (an immune complex) is detected by using fluorescence as a signal. In the EIA method, an enzyme-labeled antibody is used, an immune complex is detected by using coloring and light emission based on the enzyme reaction as a signal.

For measurement of MK, an excellent EIA method (see Japanese Patent Application Unexamined Publication No. H10-160735) and an ELISA methods (S. Ikematsu, et al. Br. J. Cancer 2000; 83) have been developed, and these methods or methods according to these methods may be employed in measurement. A person skilled in the art can easily modify a part of the conditions or procedure according to the detection purposes. The ELISA method has many advantages, for example, detection sensitivity is high, specificity is high, quantitativity is high, an operation is simple, and multiple specimens can be handled simultaneously. One example of a specific operation in which the ELISA method is used is described hereinafter. Firstly, an anti-MK antibody is immobilized to an insoluble support. Specifically, for example, the surface of a microplate is sensitized (coated) with an anti-MK antibody. A urine specimen is brought into contact with the thus solid-phased antibody. As a result of this operation, when an antigen (MK) against the solid-phased anti-MK antibody is present in the urine specimen, an immune complex is formed. Non-specific bonding components are removed by washing, followed by adding an antibody to which an enzyme is bound so as to label the immune complex. Then, the substrate of the enzyme is reacted to develop color. Thus, the immune complex is detected using an amount of color development as an indicator. Since the detail of the ELISA method is described in many text books or papers, when experiment procedures or experiment conditions of each method are set, such books or papers can be referred to. Note here that not only noncompetitive methods but also competitive methods (methods in which an antigen is added together with a specimen so as to allow them to compete with each other) may be used.

A method of directly detecting MK in a specimen with a labeled antibody may be employed or a sandwich method may be employed. In the sandwich method, two types of antibodies (a capturing antibody and a detecting antibody) whose epitopes are different from each other are used.

The time (timing) at which a urine specimen is collected is not particularly limited, but considering the fact that the MK amount is increased in an extremely early stage in patients who develop AKI, in determining the possibility of the onset of ischemic AKI that can be developed after surgeries such as an abdominal aortic aneurysm replacement surgery, a cardiac valve replacement surgery, organ transplantation, partial nephrectomy for a renal tumor, or the like, the step (1) is preferably carried out using urine collected between a procedure that may cause ischemic state in the surgery and one day after the surgery. Further preferably, the step (1) is carried out using urine collected between a procedure that may cause an ischemic state in the surgery and the end of the surgery. Most preferably, the step (1) is carried out using urine collected between the start of a procedure that may cause an ischemic state in the surgery and in three hour from the start of the surgery (the time may be during the surgery or after surgery). By using urine specimen collected in the early stage, the onset of AKI can be predicted early. The "procedure that may cause ischemic state in the surgery" herein corresponds to clamping of the blood vessel or artificial heart and lung surgery and the like. Specifically, in the case of an abdominal aortic aneurysm surgery, aortic cross clamping corresponds to the "procedure that may cause ischemic state in the surgery." Furthermore, the "end of the surgery" denotes the time when abdominal suturing procedure is carried out.

In determining the possibility of the onset of AKI after the examination using contrast media for angina pectoris, myocardial infarct, and the like, for example, urine collected in four hours after the examination using contrast media (the time when contrast media is injected is defined as reference (time 0)) is used as a specimen. Preferably, urine collected in two hours after the examination using contrast media is used as a specimen. Furthermore, in determining the possibility of the onset of AKI after drug administration, for example, urine collected in four hours after drug administration is used as a specimen. Preferably, urine collected in two hours after drug administration is used as a specimen. In the case where subjects are patients with kidney disorder (for example, patients in a group of minimal change), for example, urine collected when a patient visits to a hospital to see a doctor, when a patient is hospitalized, when a patient undergoes kidney biopsy, and the like.

In one embodiment of the present invention, not only urinary MK but also other biomarkers that may function as an indicator for AKI are used, and the detection results of the both biomarkers are used for determination of a possibility of the onset of AKI. That is to say, in this embodiment, in addition to the step (1), the following step (1') is carried out. In the step (2), the possibility of the onset of AKI is determined based on the detection results of the step (1) and step (1'):
(1') a step of detecting one or more biomarkers selected from the group consisting of urinary NGAL, urinary IL-18, urinary KIM-1, NAG and urinary L-FABP.

According to this embodiment, since determination is carried out by using two or more different indicators, examination results with high hitting rate and/or reliability can be obtained. Changes of biomarkers (including MK) have specific properties, respectively. Therefore, by combining a plurality of biomarkers, the high hitting rate and/or reliability of the possibility of the onset of AKI can be enhanced. Furthermore, useful information for estimation of prognosis, differentiation of acute tubular necrosis (ATN) and nephritis / nephropathy can be obtained.

From the viewpoint of enhancing the hitting rate and/or the reliability, in principle, the number of the biomarkers to be detected in the step (1') is as many as possible. Thus, preferably, two or more biomarkers (for example, NGAL and IL-18) are subjected to be detected in the step (1').

The measurement method of each biomarker in the step (1') is not particularly limited. For example, similar to the measurement of MK, immunoassay can be employed. The measurement of each biomarker may be carried out according to standard methods or existing methods. For example, Non-patent Document 1 describes measurement methods of NGAL (Western blotting method and ELISA method). Non-patent Document 2 describes a measurement method of NGAL (ELISA method) and a measurement method of IL-18 (ELISA method). Non-patent Document 3 describes a measurement method of KIM-1 (Western blotting method and ELISA method). Non-patent Documents 4 and 5 describe a measurement method of L-FABP (ELISA method).

The time (timing) at which a urine specimen is collected when these biomarkers are measured is not particularly limited. The time (timing) at which a urine specimen is collected may be appropriately set with considering past reports or according to the results of the preliminary experiments.

### Step (2)

In the step (2), based on the detection result of the step (1) (when the step (1') is also carried out, based on the detection results of steps (1) and (1')), the possibility of the onset of AKI is determined. The possibility of the onset may be determined qualitatively or quantitatively. Examples of the qualitative determination and quantitative determination are shown below. Note here that, as is apparent from the determination criteria, the determination herein can be carried out automatically / mechanically without depending upon the judgment by persons having special technical knowledge, for example, medical doctors or laboratory technicians.

### (Example 1 of Qualitative Determination)

When a measurement value (a MK amount) is higher than the reference value, it is determined that the "possibility of the onset is high." When a measurement value is lower than the reference value, it is determined that the "possibility of the onset is low."

### (Example 2 of Qualitative Determination)

When the reactivity is observed (positive), it is determined that the "possibility of the onset is high." When the reactivity is not observed (negative), it is determined that the "possibility of the onset is low."

### (Example of Quantitative Determination)

As shown below, the possibility of the onset (%) is previously set for each range of the measurement values, and the possibility of the onset (%) is determined from the measurement value.
Measurement values a - b: possibility of the onset is not more than 10%
Measurement values b - c: possibility of the onset is 10% to 30%
Measurement values c - d: possibility of the onset is 30% to 50%
Measurement values d - e: possibility of the onset is 50% to 70%
Measurement values e - f: possibility of the onset is 70% to 90%

One example of determination technique using other biomarkers (one or more biomarkers selected from the group consisting of urinary NGAL, urinary IL-18, urinary KIM-1, urinary NAG and urinary L-FABP) in addition to MK is described. Firstly, the possibility of the onset is determined based on the detection result of MK. When the determination result shows that the possibility of the onset is high, the detection of the other biomarkers is additionally carried out. Then, based on the detection results from the other biomarkers, the possibility of the onset is finally determined. This example employs a technique in which the detection result of MK is used for primary determination and the detection result of the other biomarker is used for final determination. On the contrary, the detection result of the other biomarkers may be used for primary determination and the detection result of MK may be used for final determination.

### 1-3. Reagent and Kit for Examination of Acute Kidney Injury (AKI)

The present invention further provides a reagent and a kit for examination of AKI. The reagent of the present invention includes an anti-MK antibody. Kinds or origins of the anti-MK antibodies are not particularly limited as long as the anti-MK antibody has a specific binding activity with respect to MK. Furthermore, a polyclonal antibody, an oligoclonal antibody (a mixture of several to several tens of kinds of antibodies), and a monoclonal antibody may be employed. As the polyclonal antibody or the oligoclonal antibody, in addition to an antiserum-derived IgG fraction obtained by immunizing animals, an affinity purified antibody by antigen can be used. The anti-MK antibody may be antibody fragments such as Fab, Fab', F(ab')₂, scFv, dsFv antibodies.

The anti-MK antibody can be prepared by using an immunologic technique, a phage display technique, a ribosome display method, and the like. The preparation of polyclonal antibody by the immunologic technique can be carried out by the following procedure. An antigen (MK or a part thereof) is prepared, and this is used to immunize an animal such as a rabbit. By purifying the living body sample, an antigen can be obtained. A recombinant antigen can be also used. The recombinant MKcan be prepared by introducing a gene encoding MK (a part of the gene may be used) into a suitable host by using a vector to obtain a recombinant cell, and expressing the gene in the obtained recombinant in the cell.

In order to strengthen the immune elicitation, an antigen to which a carrier protein is bonded may be used. As the carrier protein, KLH (Keyhole Limpet Hemocyanin), BSA (Bovine Serum Albumin), OVA (Ovalbumin), and the like, are used. For bonding of the carrier protein, a carbodiimide method, a glutaraldehyde method, a diazo condensation method, a MBS (maleimidobenzoyloxy succinimide) method, and the like, can be used. On the other hand, it is possible to use an antigen in which antigen (or a part thereof) is allowed to be expressed as a fusion protein with GST, β galactosidase, maltose binding protein, or histidine (His) tag, and the like. Such a fusion protein can be purified by a general method in a simple and easy manner.

Immunization is repeated if necessary. At a time when an antibody value is sufficiently increased, blood is collected. The collected blood is subjected to centrifugation so as to obtain serum. The obtained anti-serum is subjected to affinity purification to give a polyclonal antibody.

On the other hand, the monoclonal antibody can be prepared by the following procedure. Firstly, immunization is carried out by the same procedure as mentioned above. Immunization is repeated if necessary. At the time when an antibody value is sufficiently increased, an antibody producing cell is extracted from an immunized animal. Next, the obtained antibody producing cell and myeloma cell are fused so as to obtain hybridoma. Subsequently, this hybridoma is made to be monoclonal. Clones capable of producing an antibody having high specificity with respect to the objective protein are selected. By purifying a culture solution of the selected clone, the objective antibody can be obtained. On the other hand, the hybridoma is allowed to proliferate to the predetermined number or more. Then, the proliferated hybridoma are transplanted into the abdominal cavity of an animal (for example, a mouse) and allowed to proliferate in the abdominal dropsy. By purifying the abdominal dropsy, the objective antibody can be obtained. For purification of the above-mentioned culture solution or purification of the abdominal dropsy, affinity chromatography using protein G, protein A, and the like, can be suitably used. Furthermore, affinity chromatography in which the antigen is immobilized to a solid phase can be used. Furthermore, methods such as ion exchange chromatography, gel filtration chromatography, ammonium sulfate fractionation, and centrifugation can be also used. These methods may be used singly or may be used in combination thereof.

Various modifications can be made to the obtained antibody on the condition that specific connectivity is maintained. Such a modified antibody may be included in the reagent of the present invention.

When a labeled antibody is used as the anti-MK antibody, a binding amount of the antibody can be directly detected by using a labeling amount as an indicator. Therefore, a simpler and easier examination method can be established. However, there are problems that it is necessary to prepare an anti-MK antibody to which a labeling substance is attached, and furthermore, the detecting sensitivity is generally lowered. Then, it is preferable to use indirect detection methods such as a method using a secondary antibody to which a labeling substance is attached and a method using a secondary antibody and a polymer to which a labeling substance is bound. The secondary antibody herein denotes an antibody having a specific binding activity to the anti-MK antibody. For example, when the anti-MK antibody is prepared as a rabbit antibody, an anti-rabbit IgG antibody can be used. Labeling secondary antibodies that can be used in various species such as rabbit, goat and mouse are available (for example, products available from Funakoshi Corporation, COSMO BIO Co., Ltd.), and appropriate antibodies can be selected and used according to the reagent of the present invention.

Examples of the labeling substances include enzymes such as peroxidase, micro-peroxidase, horseradish peroxidase (HRP), alkaline phosphatase, β-D-galactosidase, glucose oxidase, and glucose-6-phosphate dehydrogenase; fluorescence substances such as fluorescein isothiocyanate (FITC), tetramethylrhodamine isothiocyanate (TRITC), and europium; chemiluminescence substances such as luminol, isoluminol and acridinium derivative; coenzymes such as NAD; biotin; as well as radioactive substances such as ¹³¹I and ¹²⁵I.

In one embodiment, the reagent of the present invention is immobilized to a solid phase in accordance with the use. Insoluble supports to be used for immobilization into a solid phase are not particularly limited. For example, an insoluble support made of a substance insoluble in water, for example, resin such as polystyrene resin, polycarbonate resin, silicon resin, and nylon resin, glass, and the like can be used. An antibody can be supported to an insoluble support by physical adsorption or chemical adsorption.

The kit of the present invention includes the reagent of the present invention as a main component. The kit may include other reagents (buffer solution, blocking reagent, substrate for enzyme, coloring reagent, and the like) used for carrying out a examination method and/or a device or an instrument (a container, a reactor, a fluorescence reader, and the like). Furthermore, it is preferable that the kit includes MK as a standard sample. Furthermore, the kit may include a reagent for detecting biomarkers other than MK (one or more biomarkers selected from the group consisting of urinary NGAL, urinary IL-18, urinary KIM-1, urinary NAG and urinary L-FABP), other reagents necessary for using the reagent, an instrument, and the like. In general, an instruction manual is attached to the kit of the present invention.

### 2. Estimation of Prognosis associated with Renal Function

An examplatory embodiment relates to estimation of prognosis associated with a renal function. In this example, a biomarker for estimation of prognosis associated with a renal function and use thereof are provided. Hereinafter, this example is described in detail.

### 2-1. Biomarker for Estimation of Prognosis Associated with Renal Function

The "biomarker for estimation of prognosis associated with a renal function" in this specification denotes a biomolecule used as an indicator for estimation of prognosis associated with a renal function. The "prognosis associated with a renal function" is a future state of a renal function, and includes a state of a future renal function of a patient undergoing an event that may cause renal dysfunction and a state of a future renal function of a patient with renal dysfunction. The "event that may cause renal dysfunction" includes all of ischemic AKI, toxic AKI, and septic AKI. Examples of the ischemic AKI include AKI developed after surgeries, for example, a surgery of a large blood vessel such as an abdominal aortic aneurysm replacement surgery, open heart surgery such as cardiac valve replacement surgery using an artificial heart lung machine, organ transplantation, or partial nephrectomy of a renal tumor; AKI developed accompanying congestive heart failure, administration of contrast media (for example, examination using contrast media for angina pectoris, myocardial infarct, and the like), hepatorenal syndrome or thrombotic microangiopathy; AKI caused by drugs such as calcineurin inhibitor, angiotensin converting enzyme inhibitor, angiotensin II receptor antagonist,non-steroidal anti-inflammatory analgesic, and the like. However, the AKI is not necessarily limited to these examples. On the other hand, examples of the toxic AKI include AKI caused by exogenous factors such as drugs, for example, an antibacterial agent and an anticancer drug, and endogenous factors such as heme pigment (hemoglobinuria, myoglobinuria), abnormal protein in, for example, multiple myeloma, crystalline components such as uric acid and methotrexate, and the like.

### 2-2. Estimation Method of Prognosis Associated with Renal Function

Reference is made to a method for estimating prognosis associated with a renal function as use of the biomarker thereof. The method for estimating prognosis uses an amount of urinary MK as a determination indicator, and includes a step (step (1)) for detecting urinary MK and a step (step (2)) for estimating the prognosis based on the detection result. Since the step (1) is the same as the step (1) in the first aspect, the description thereof is omitted herein. Note here that urine derived from the affected kidney and/or urine derived from the healthy kidney are used as specimens.

The subsequent step (2) estimates prognosis by using the amount of urinary MK as an indicator. For example, when the postoperative prognosis of surgeries, for example, a surgery of a large a large blood vessel such as an abdominal aortic aneurysm replacement surgery, open heart surgery such as cardiac valve replacement surgery using an artificial heart lung machine, organ transplantation, or partial nephrectomy, is estimated, the prognosis can be estimated according to the following criterion.

### (Determination Criterion)

The increase of the amount of urinary midkine observed from six hours after the surgery to 48 hours after the surgery is transitional, the prognosis is excellent, and the increase of the amount of urinary midkine observed from six hours after the surgery to 48 hours after the surgery is not transitional while the increased value is protracted or maintained, the prognosis is poor.

Note here that, as is apparent from the determination criteria, the determination herein can be carried out automatically / mechanically without depending upon the judgment by persons having special technical knowledge, for example, medical doctors or laboratory technicians.

### 2-3. Reagent and Kit for Estimation of Prognosis Associated with Renal Function

Reference is further made to a reagent and a kit for estimation of prognosis associated with a renal function. The reagent includes an anti-MK antibody. The anti-MK antibody is the same as the case of the reagent of the first aspect, the description thereof is omitted herein. The kit includes the reagent as a main component. The kit may include other reagents (a buffer solution, a blocking reagent, a substrate for enzyme, a coloring reagent, and the like) and/or a device or an instrument (a container, a reactor, a fluorescence reader, and the like) used for carrying out an estimation method. Furthermore, it is preferable that the kit includes MK as a standard sample. Note here that an instruction manual is attached to the kit of the present invention.

### 3. Differential Diagnosis of Acute Kidney Injury (AKI)

A further examplatory embodiment relates to differential diagnosis of AKI. This example provides a biomarker for differentiation of the kidney with acute kidney injury and use thereof. Hereinafter, this example is described in detail.

### 3-1. Biomarker for Differentiation of Kidney with Acute Kidney Injury

The "biomarker for differentiation of the kidney with acute kidney injury (AKI)" in this specification denotes a biomolecule used as an indicator for differentiation of AKI (determination of whether or not the case of AKI is acute tubular disorder represented by acute tubular necrosis (ATN)).

### 3-2. Differentiation Method of Acute Kidney Injury (AKI)

Reference is also made to a differentiation method of AKI as one of uses of the biomarker of the. The differentiation method of the permits determination of whether or not the cause of AKI is acute tubular disorder represented by acute tubular necrosis (ATN). Typically, determination results about the cause of AKI: "the cause is ATN" or "the cause is kidney disorder other than ATN" are obtained.

In the differentiation method, the amount of urinary MK is used as a determination indicator. Specifically, the differentiation includes the following steps (1) and (2).
(1) detecting urinary MK;
(2) determining whether or not the cause of acute kidney injury is acute tubular disorder based on the detection result.

Since the step (1) is the same as the step (1) in the first aspect of the present invention, the description thereof is omitted herein.

In one embodiment, not only urinary MK but also other biomarkers that function as indicators of AKI are detected, and the detection results of both indicators are used for differentiation of AKI. That is to say, in this embodiment, in addition to the step (1), the following step (1') is carried out. In the step (2), determination of whether or not the cause of acute kidney injury is acute tubular disorder based on the detection results of the steps (1) and (1') is carried out:
(1') a step of detecting one or more biomarkers selected from the group consisting of urinary NGAL, urinary IL-18, urinary KIM-1, NAG, and urinary L-FABP.

According to this embodiment, since determination is carried out by using two or more different indicators, differentiation results with high hitting rate and/or reliability can be obtained. Changes in biomarkers (including MK) have specific properties, respectively. Therefore, by combining a plurality of biomarkers, the high hitting rate and/or reliability of the differentiation results can be enhanced. Furthermore, useful information for estimation of prognosis can be obtained.

From the viewpoint of enhancing the hitting rate and/or the reliability, in principle, the number of the biomarkers to be detected in the step (1') is as many as possible. Thus, preferably, two or more biomarkers (for example, NGAL and IL-18) are subjected to be detected in the step (1').

The measurement method of each biomarker in the step (1') is not particularly limited. Standard methods and existing methods of each biomarker are described in the first aspect.

### Step (2)

In the step (2), based on the detection result of the step (1) (when the step (1') is also carried out, based on the detection results of steps (1) and (1')), it is determined whether or not the cause of AKI is acute tubular disorder. The determination herein may be qualitative determination or quantitative determination. Examples of the qualitative determination and quantitative determination are shown below. Note here that, as is apparent from the determination criteria, the determination herein can be carried out automatically / mechanically without depending upon the judgment by persons having special technical knowledge, for example, medical doctors or laboratory technicians.

### (Example 1 of Qualitative Determination)

When a measurement value (amount of MK) is larger than the reference value, it is determined that "the cause is ATN" or "there is a high possibility that the cause is ATN." When a measurement value (amount of MK) is smaller than the reference value, it is determined that "the cause is not ATN" or "there is a high possibility that the cause is not ATN." When a measurement value is smaller than the reference value, it can be determined that "the cause is kidney disorder other than ATN" or "there is a high possibility that the cause is kidney disorder other than ATN."

### (Example 2 of Qualitative Determination)

When the reactivity is observed (positive), it is determined that "the cause is ATN" or "there is a high possibility that the cause is ATN." When the reactivity is not observed (negative), it is determined that "the cause is not ATN" or "there is a high possibility that the cause is not ATN" or "the cause is kidney disorder other than ATN" or "there is a high possibility that the cause is kidney disorder other than ATN."

### (Example of Quantitative Determination)

As shown below, "the possibility that the cause is ATN (%)" is previously set for each range of the measurement value, and "the possibility that the cause is ATN (%)" is determined from measurement value.
Measurement values a - b: possibility that the cause is ATN is not more than 10%
Measurement values b - c: possibility that the cause is ATN is 10% to 30%
Measurement values c-d: possibility that the cause is ATN is 30% to 50%
Measurement values d - e: possibility that the cause is ATN is 50% to 70%
Measurement values e - f: possibility that the cause is ATN is 70% to 90%

One example of determination technique using the other biomarkers (one or more biomarkers selected from the group consisting of urinary NGAL, urinary IL-18, urinary KIM-1, urinary NAG and urinary L-FABP) in addition to MK is described. Firstly, the possibility that the cause is ATN is determined based on the detection result of MK. When the determination result shows that the possibility that the cause is ATN is high, the detection of other biomarkers is additionally carried out. Then, based on the detection results of the other biomarkers, the final determination that the possibility that the cause is ATN is made. This example employs a technique in which the detection result of MK is used for the primary differentiation and the detection result of other biomarkers is used for the final differentiation. On the contrary, the detection result of the other biomarkers may be used for the primary differentiation and the detection result of MK may be used for the final differentiation.

When two or more biomarkers are used in combination, the following determination method (1) or (2) may be employed.
(1) When all of the biomarkers included in the combination are positive (not less than a cut-off value), the result is determined to be positive, and if it is not the case, the result is determined to be negative.
(2) When at least one of the biomarkers included in the combination is positive (not less than a cut-off value), the result is determined to be positive, if it is not the case, the result is determined to be negative.

According to the study of the present inventors, it is shown that the diagnosis sensitivity and the diagnosis specificity are different depending on the kinds and the number of the biomarkers to be combined (see the below-mentioned Examples). Therefore, the suitable combination of the biomarkers may be selected. For example, the combination in which the diagnosis sensitivity is high is suitable for screening examinations. On the contrary, combination in which the diagnosis specificity is high is suitable for examinations that require high reliability (for example, secondary examination and tertiary examination).

By combining the determination methods by varying the balance between the diagnosis sensitivity and the diagnosis specificity, it is possible to improve efficiency, accuracy or reliability. For example, after the combination of biomarkers giving high diagnosis sensitivity is used so as to narrow down the positive subjects (primary examination, screening examination), the combination of biomarkers giving high diagnosis specificity is used so as to make the final determination (secondary examination). Not only such a two-step determination but also three-step determination can be carried out.

Examples of the combinations of biomarkers include a combinations of MK and NAG, a combinations of MK and IL-18, a combinations of MK and NGAL, a combinations of MK, NAG and IL-18, a combinations of MK, NAG and NGAL, a combinations of MK, IL-18 and NGAL, as well as a combinations of MK, NAG, IL-18 and NGAL. As shown in the below-mentioned Examples, it is expected that such combinations improve the diagnosis specificity except for the combinations of MK and NGAL (in this case, result showing a good balance of the sensitivity and specificity is obtained).

### 3-3. Reagent and Kit for Differentiation of Acute Kidney Injury (AKI)

Reference is further made to a reagent and a kit for differentiation of AKI. The reagent of the includes an anti-MK antibody. Since the anti-MK antibody is the same as in the reagent of the first aspect of the present invention, the description thereof is omitted. The kit includes the reagent as a main component. The kit may include other reagents (buffer solution, blocking reagent, substrate for enzyme, coloring reagent, and the like) and/or a device or instrument (container, reactor, fluorescence reader, and the like), which are used for carrying out a differentiation method. Furthermore, it is preferable that the kit includes MK as a standard sample. Note here that an instruction manual is attached to the kit.

### [Example]

### 1. Urinary Midkine (MK) Concentration in Various Renal Diseases

### (1) Subjects

MK is a heparin-binding growth factor, and its expression is strengthened in the proximal tubule in ischemic disorder (Sato W. et al., J Immunol. 2001 Sep 15; 167(6): 3463-9). The relation between the urinary MK concentration and AKI was examined in 583 subjects. The 583 subjects include 33 patients with acute tubular necrosis (ATN) that is the main cause of AKI, 517 patients with renal diseases other than AKI, and 33 healthy volunteers.

### (2) Methods

After written consent was obtained, urine of each subject was collected, and the urinary MK concentration was measured. Specifically, by using 0.01 ml of urine specimen, the urinary MK concentration was measured by sandwich ELISA using two kinds of polyclonal anti-MK antibodies (a chicken antibody and a rabbit antibody).

### (3) Results

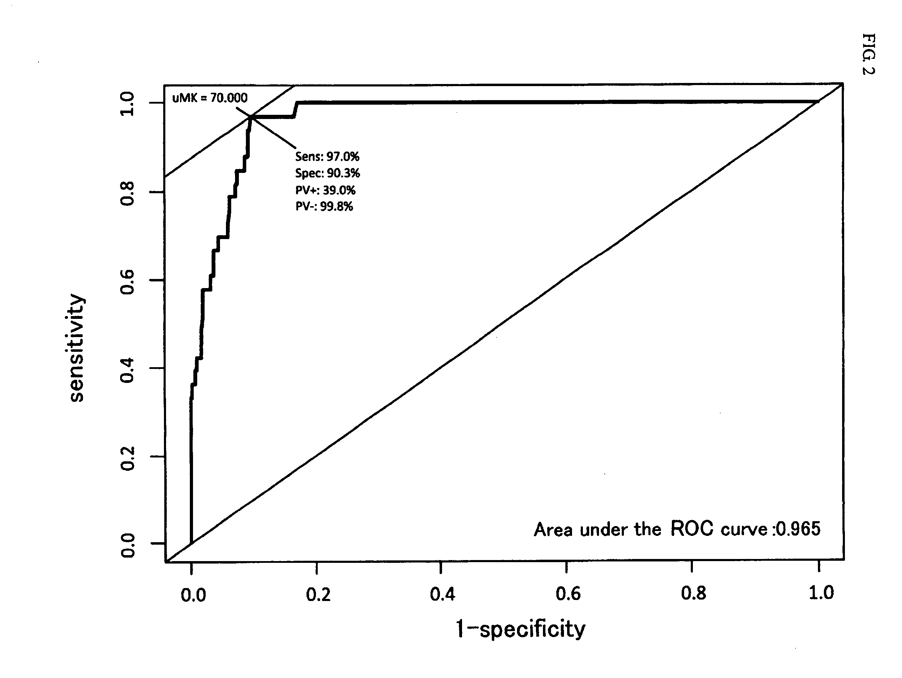

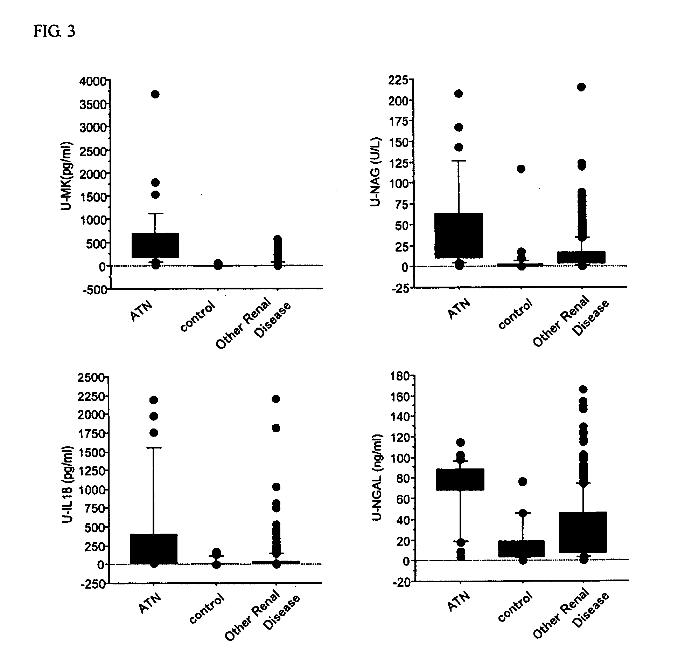

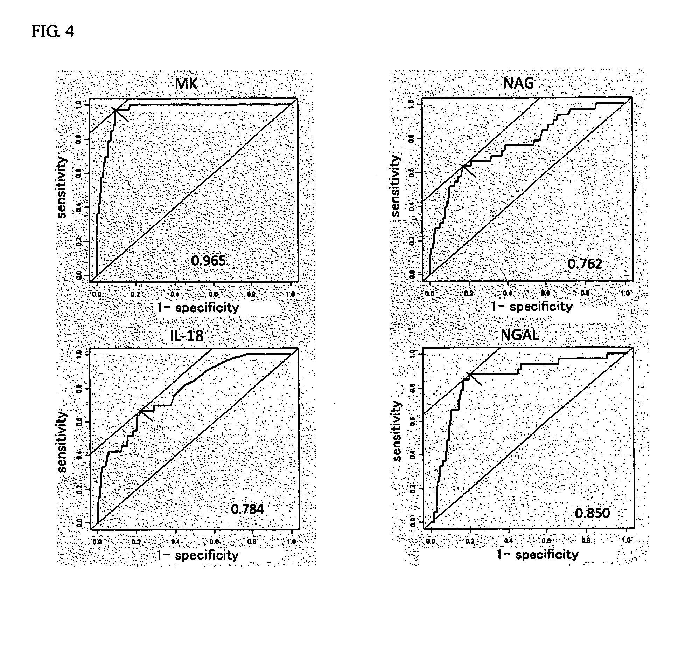

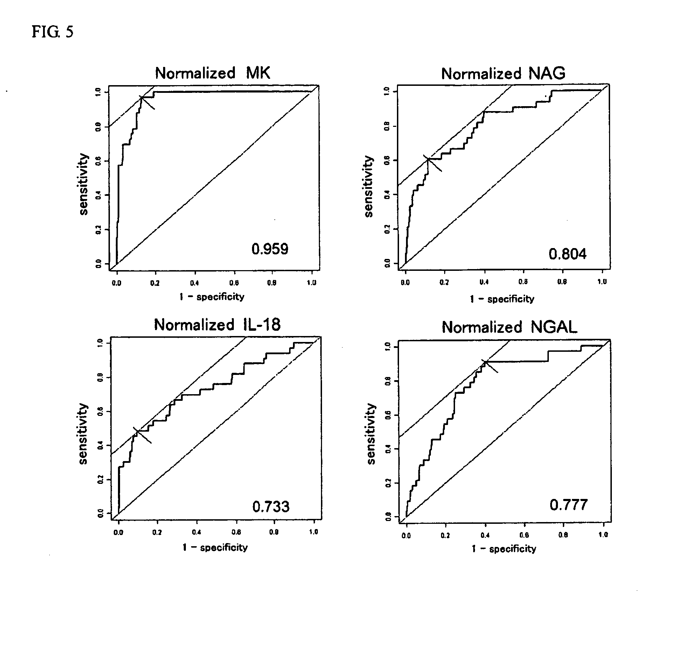

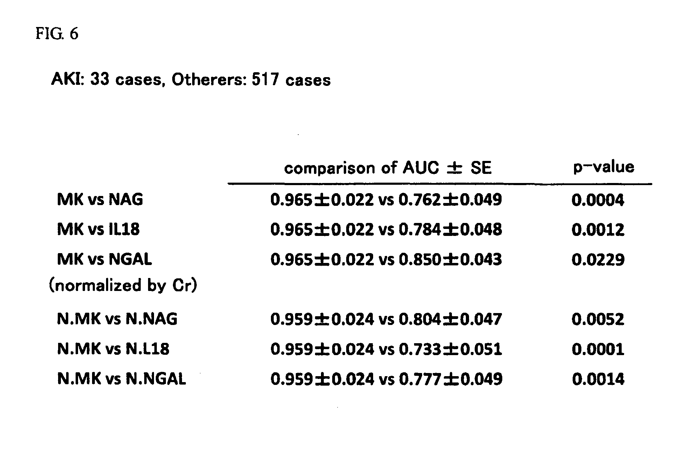

The measurement result (actual measurement value) of the urinary MK concentration is shown in Fig. 1. The result of the ROC curve (receiver-operating characteristic curve) analysis is shown in Fig. 2. As compared with the urinary MK concentrations of the control group or patients with renal diseases other than AKI, significant increase in the urinary MK concentration was observed in patients with acute tubular necrosis (ATN) that is a main cause of AKI (Fig. 1). AUC (area under the curve) of the ROC curve as an indicator for the sensitivity and specificity of biomarker was 0.965, which was a especially excellent result for a single biomarker (Fig. 2). In the existing urinary biomarkers (β-N-acetyl-D-glucosaminidase (NAG), urinary neutrophil gelatinase-associated lipocalin (NGAL), and urinary interleukin 18 (IL-18)), the concentration change specific to AKI as in the urinary MK is not observed (Fig. 3). As shown in Fig. 4 (comparison of actual measurement values), Fig. 5 (comparison of values normalized by the urinary creatinine (Cr)), and Fig. 6 (summary of comparisons), AUC of the urinary MK concentration is significantly high as compared with those of urinary NAG, urinary IL-18, and urinary NGAL. When the cut-off value of the urinary MK concentration was set to 70 pg/ml, the sensitivity and the specificity in ATN diagnosis using the urinary MK concentration were 97.0% and 90.3%, respectively, which were superior to those of the existing urinary biomarkers (NAG, NGAL, and IL-18).

As mentioned above, it has been shown that the urinary MK concentration is a sensitive biomarker for AKI, and it exhibits more usefulness than the existing biomarker. That is to say, it can be said that the urinary MK concentration is the most excellent biomarker in differential diagnosis of AKI.

By the way, although most of the cases of acute kidney injury are ischemic AKI, the cases also include other categories such as septic AKI, toxic AKI accompanying administration of anti-cancer drugs, and radiocontrast nephropathy (which may be included in ischemic AKI). As shown below, also in these acute kidney injury cases, an increase in the urinary MK concentration was observed.
Septic AKI: 710 pg/ml (female patient, age 38), 750 pg/ml (male patient, age 75), 502 pg/ml (male patient, age 74)
Toxic AKI: 141 pg/ml (female patient, age 13)
Radiocontrast nephropathy: 272 pg/ml (male patient, age 41)

### 2. AKI Diagnosis Using Combination of Biomarkers

AKI diagnosis was carried out by using a combination of the urinary MK concentration and other biomarkers, and the sensitivity and specificity were calculated. As the value of each biomarker, values normalized by Cr (corresponding to the ROC curve shown in Fig. 5) were used. Furthermore, by the analysis of the ROC curve, the cut-off value of each biomarker was set as follows.
Cut-off value of urinary MK concentration: 75.1 pg/mgCr
Cut-off value of urinary NAG concentration: 31.0 U/gCr
Cut-off value of urinary IL-18 concentration: 173.6 pg/mgCr
Cut-off value of urinary NGAL concentration: 26.7 ng/mgCr

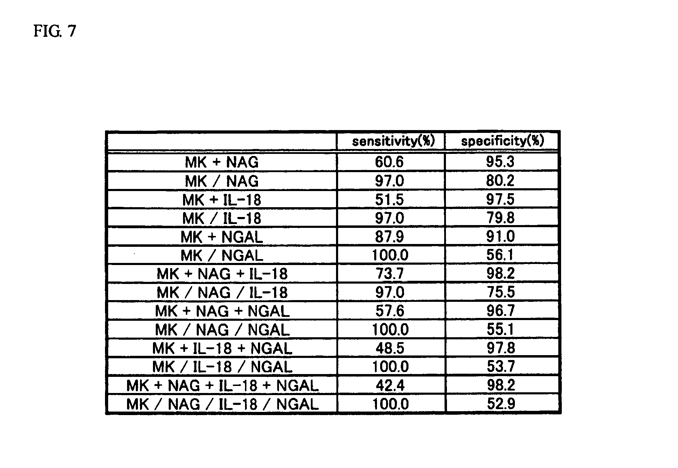

Various combinations were subjected to statistical processing, and comparisons of the sensitivity and the specificity were carried out (Table of Fig. 7). In the case in which the combination represented by a mark "+," when all biomarkers included in the combination were positive (not less than a cut-off value), the result was determined to be positive, and the sensitivity and the specificity at this time were calculated. For example, in MK + IL-18 + NGAL, when all of the urinary MK concentration, urinary IL-18 concentration and urinary NGAL concentration are positive (not less than a cut-off value), the result is determined to be positive, and if it is not the case, the result is determined to negative. The sensitivity (48.5%) and the specificity (97.8%) at this time are shown. On the other hand, in the case in which the combination represented by a mark "/," when at least one of the biomarkers included in the combination was positive (not less than a cut-off value), the result was determined to be positive, and the sensitivity and the specificity at this time were calculated. For example, in MK/IL-18/NGAL, at least one of the urinary MK concentration, urinary IL-18 concentration and urinary NGAL concentration is positive (not less than a cut-off value), the result is determined to be positive, and if it is not the case, the result is determined to be negative. The sensitivity (100%) and the specificity (53.2%) at this time are shown.

As shown in the Table of Fig. 7, depending upon the kinds and number of the biomarkers to be combined, balance of the sensitivity and the specificity varies. This means that by selecting the combination of biomarkers according to purposes, diagnosis corresponding to the purposes can be carried out. In general, the combination in which sensitivity is high (for example, MK/NGAL, MK/IL-18/NGAL, or MK/NAG/IL-18/NGAL) is suitable for screening examination. On the contrary, combination in which specificity is high (for example, MK + NAG, MK + IL-18, MK + NAG + IL-18, MK + NAG + NGAL, MK + IL-18 + NGAL, or MK + NAG + IL-18 + NGAL) is suitable for examinations that require high reliability, for example, secondary examination and tertiary examination. For example, when an examination using the former combination is carried out so as to narrow down positive subjects, and then an examination using the latter combination is carried out to make a final determination, it is possible to carry out AKI diagnosis (differentiation of AKI) efficiently and with high reliability.

### 3. Acute Kidney Injury (AKI) after Abdominal Aortic Aneurysm Surgery and Urinary Midkine (MK) Concentration

### (1) Subjects

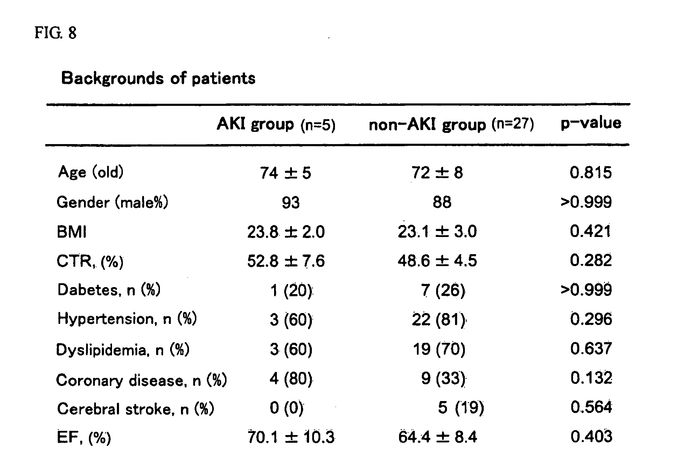

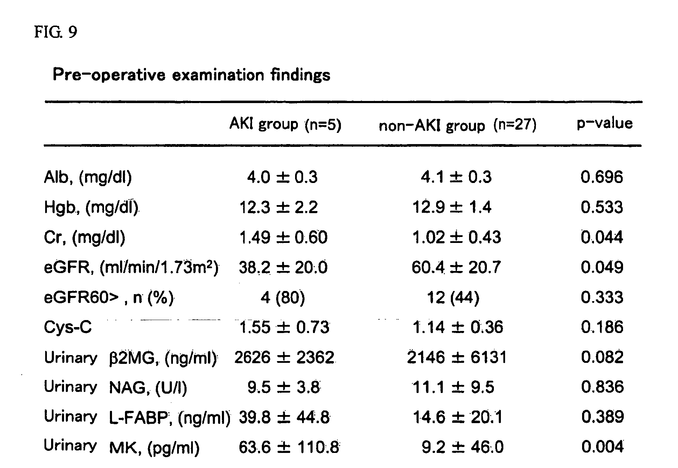

Subjects were 32 patients who underwent a palliative surgery of abdominal aortic aneurysm from December 2005 to May 2007 in Nagoya University Hospital and who submitted written consent. For the subjects, the relation between the urinary MK concentration and postoperative AKI was examined. Details of the backgrounds of the patients are shown in Fig. 8 and preoperative examination findings are shown in Fig. 9, respectively.
Sex: 29 male patients, 3 female patients
Age: 72 ± 8
Type of disease: upper renal artery: 2 cases, lower renal artery: 30 cases
Artificial blood vessel: type I: 11 cases, type Y: 21 cases
Estimated GFR: 56.9 ± 21.8 (ml/min/1.73m²)

### (2) Methods

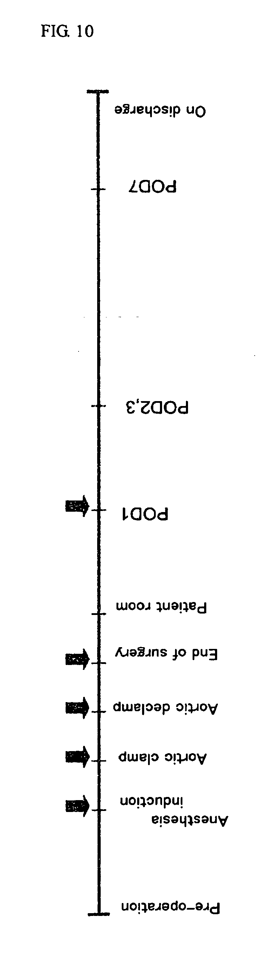

A prospective observation examination was carried out. The urinary MK concentration was measured by ELISA over time along with a general examination (S. Ikematsu, et al. Br. J. Cancer 2000; 83) (see Fig. 10). An AKI group (defined by ΔCr ≥ 50%) and a non-AKI group were compared and evaluated.

### (3) Results

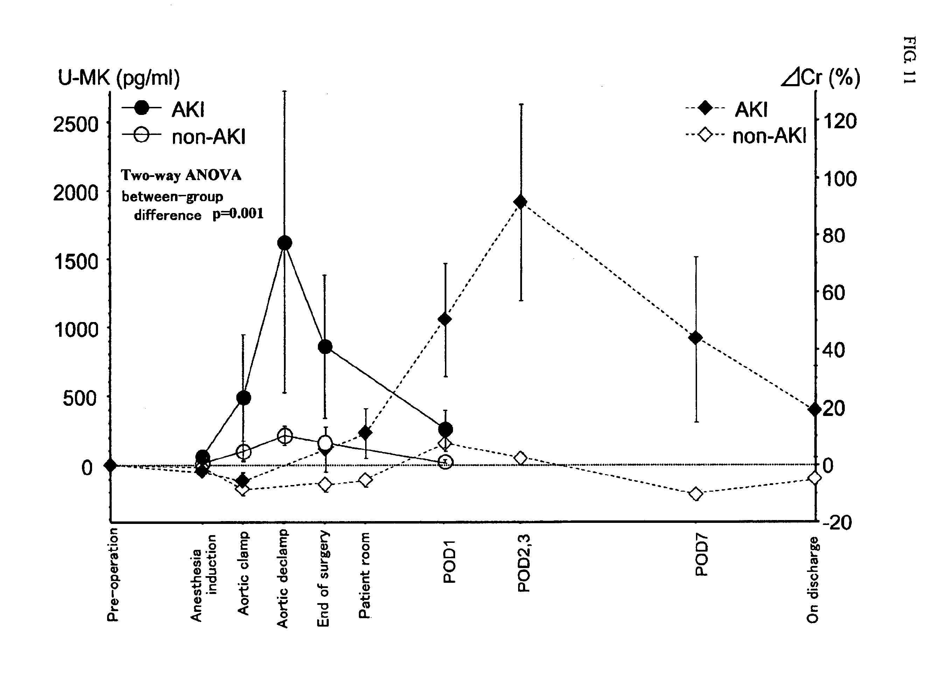

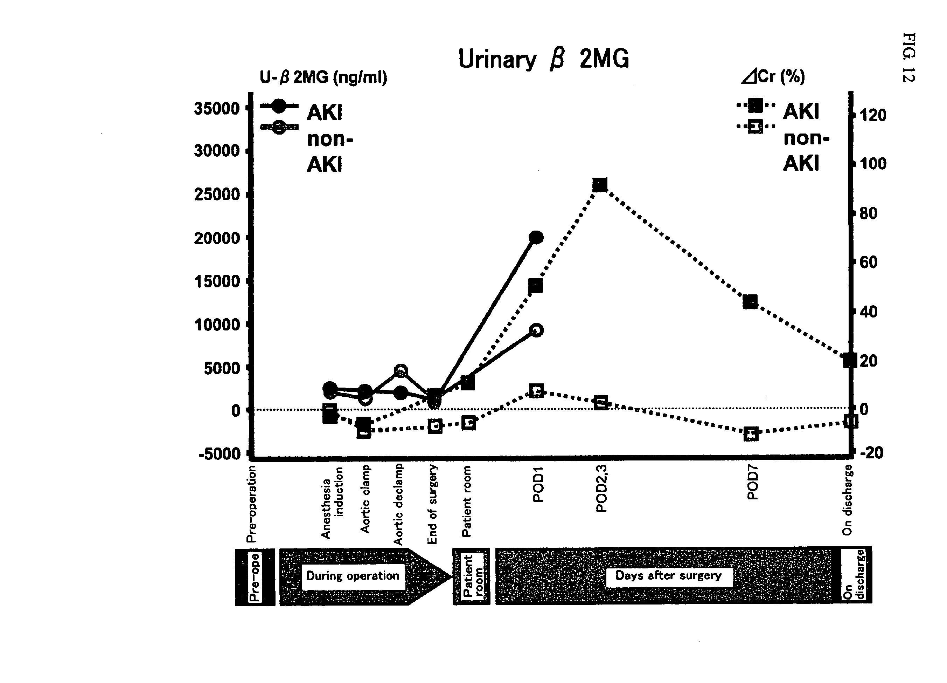

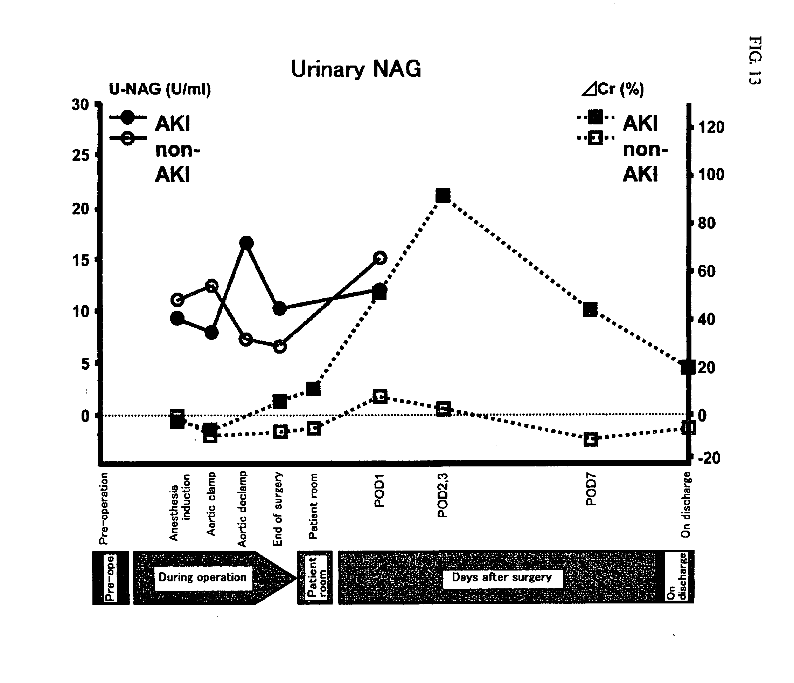

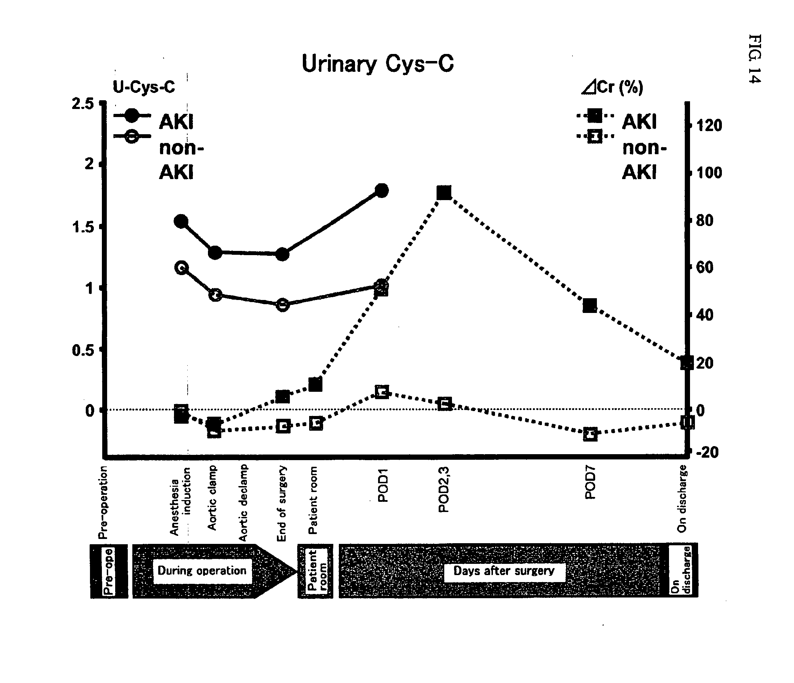

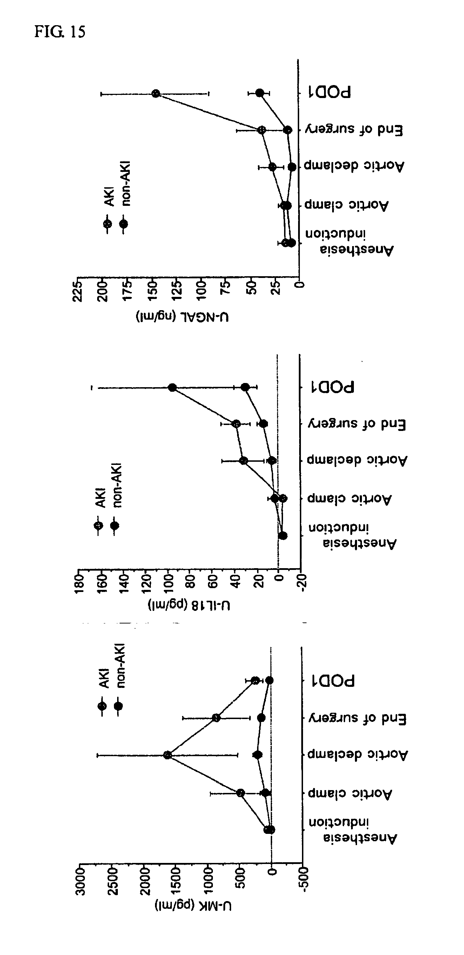

Examination results are shown in Fig. 11. From the increase in Cr having a peak on day 2 after the operation (POD2) and on day 3 after the operation (POD3), five cases (16%) were diagnosed to be AKI. In the AKI group, prior to Cr, the urinary MK concentration was increased in real time (Two-way ANOVA between-group difference, p = 0.001), and showed the high value from the time of aortic cross clamping to on day 1 after the operation (POD1). In particular, from the time of aortic cross clamping to the end of the surgery (about three hours after the aortic cross clamping), an extremely high value was shown (peak was the time of aorta opening about one hour after the time of aortic cross clamping).
From the above-mentioned results, it has been revealed that the cases in which the urinary MK concentration is increased during the surgery has extremely high possibility that postoperative AKI develops. In other words, it has been proved that when the urinary MK is used as a marker, the prediction of the onset of AKI can be carried out in extremely early stage such as during surgery. On the other hand, when comparison with the existing markers such as urinary NAG, urinary β2 microglobulin (β2 MG), and urinary cystatin C (Cys-C) was carried out, such markers did not allow early prediction of the onset (Figs. 12 to 14). Furthermore, the concentrations of urinary IL-18 and urinary NGAL reported to be early diagnostic markers for acute kidney injury, the increase was observed after surgery (Fig. 15). Thus, with the existing biomarkers (including biomarkers covered by insurance), it is impossible to carry out early prediction as in the case in which the urinary MK concentration is used. Note here that urinary NAG, and urinary β2 microglobulin were measured in an external institution. Furthermore, serum cystatin C, urinary IL-18 and urinary NGAL were measured by using commercially available ELISA measurement kit (for urinary cystatin C, Mescoat GC cystatin C kit (gold colloid aggregation method, available from Alfresa Pharma Corporation) was used; for urinary NGAL, NGAL ELISA Kit (CircuLex NGAL/Lipocalin-2 ELISA Kit/Cat#CY-8070/CircuLex) was used; for urinary IL-18, Human IL-18 ELISA Kit (CODE No 7620, MBL) was used respectively by using 0.01 ml of urine specimen.

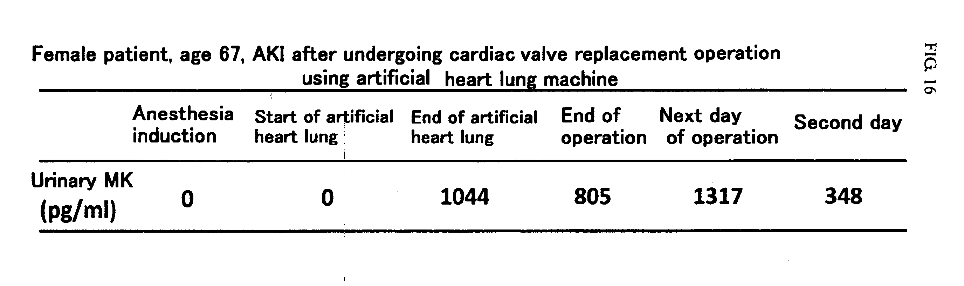

Note here that also in the cases in which AKI developed after a cardiac valve replacement surgery using artificial heart lung machine was carried out, significant increase in the urinary MK concentration was observed during surgery (Fig. 16). Thus, results supporting usefulness of the urinary MK in early prediction of the onset of AKI was obtained.

### 4. Renal Function of Transplanted Kidney after Kidney Transplantation and Urinary Midkine (MK) Concentration

### (1) Subjects and Methods

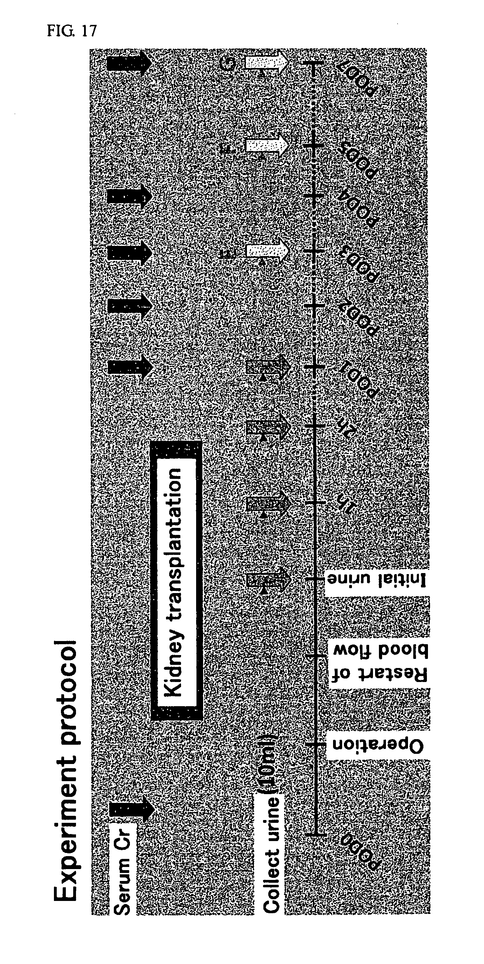

In two cases undergoing living donor renal transplantation, as shown in the protocol of Fig. 17, urine was collected over time after blood flow was restarted to graft (0 hour), at the time of initial urine, after one hour, after two hours, after six hours, after eight hours and for several days thereafter, and the concentrations of blood creatinine and urinary MK were measured.

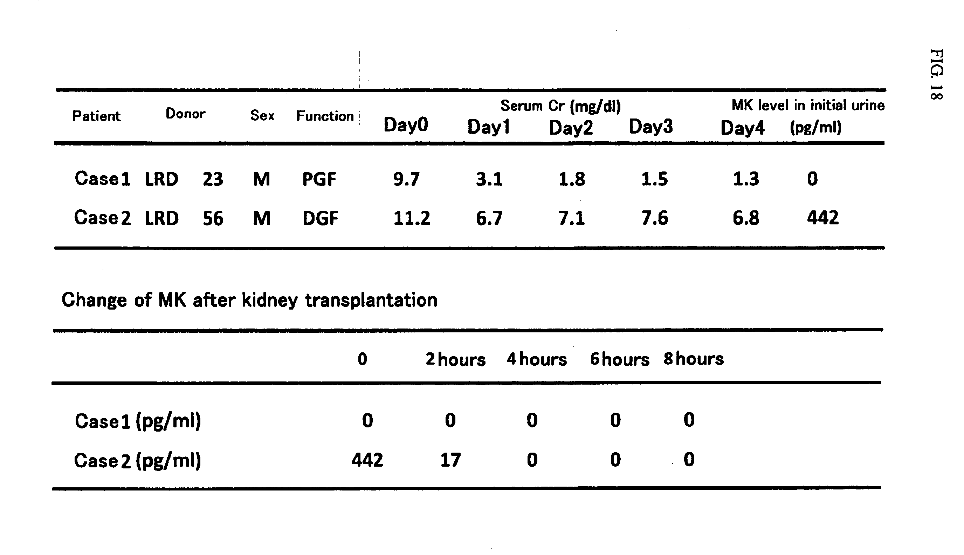

### (2) Results (Fig. 18)

In the case 1, survival after transplantation was good, and the serum creatinine was recovered favorably from the following day. In this case, urinary MK was 0 pg/ml from the time immediately after transplantation to eight hours after the start of diuresis. On the contrary, in the case 2, survival of the transplanted kidney after transplantation was bad. Until day 4 after the transplantation, a renal failure state was protracted. The urinary MK concentration of this patient was high as 442 pg/ml immediately after the start of diuresis, then reduced, and after four hours or later, the concentration was under sensitivity.

### (3) Discussion

By measuring the concentration of urinary MK immediately after the start of diuresis, it is possible to predict whether or not survival of the transplanted kidney is good (very early diagnostic agent of acute kidney injury (AKI)). In particular, in foreign countries, since many cases of cadaver kidney are carried out, bad survival is protracted after surgery in many cases. In these cases, by predicting whether or not the survival is bad immediately after the surgery, treatment for the survival of transplanted kidney is changed to more active treatment method from a general protocol, and thus, the survival rate of the transplanted kidney can be increased.

### 5. Renal Diseases due to Contrast Media and Urinary Midkine (MK) Concentration

### (1) Subjects and methods

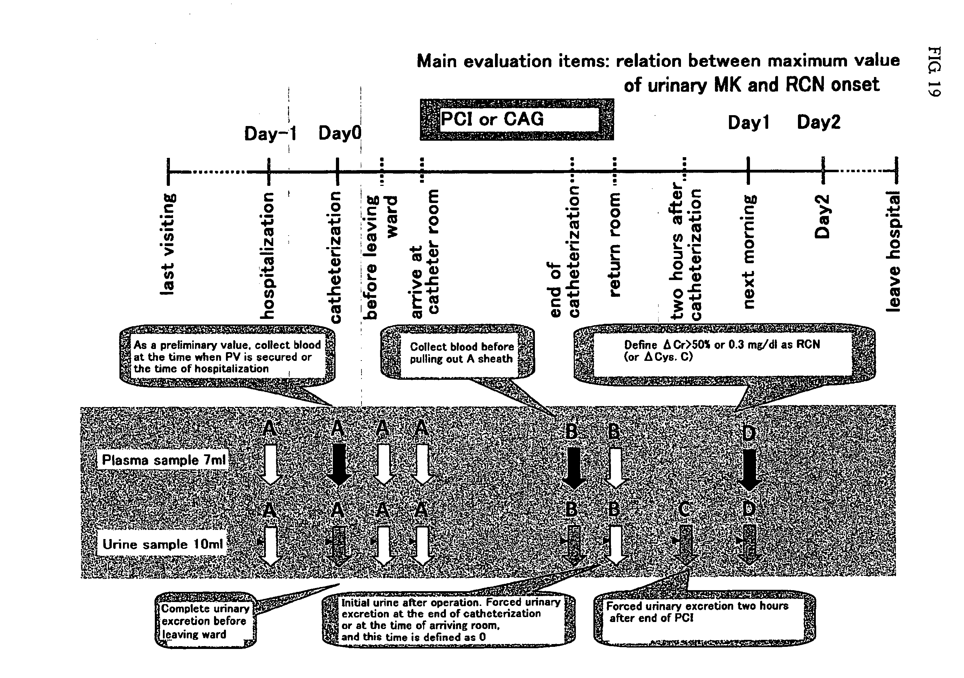

Examinations using contrast media are widely used in daily medical care, but renal diseases due to contrast media pose large clinical problem, thus increasing the necessity of biomarkers capable of early diagnosing. Among them, there are a large number of cases of coronary angiography and coronary angioplasty of angina pectoris and myocardial infarct. As shown in the protocol of Fig. 19, collection of blood and collection of urine were carried out immediately before and after, and following day of the cardiac angiography, and serum creatinine, urinary MK concentration, urinary IL-18 concentration and urinary NGAL concentration were measured.

### (2) Results

Immediately after the use of contrast media for patients with chronic renal diseases (serum creatinine was 7.62 mg/dl), the urinary MK concentration, urinary IL-18 concentration and urinary NGAL concentration in the initial urine were high as 272 pg/ml, 18.69 pg/ml and 68.88 ng/ml, respectively.

### (3) Discussion

The cardiac angiography is a diagnosis and treatment method carried out all over the world. It is shown that the measurement of the urinary MK immediately after the examination makes it possible to predict contrast media kidney injury (toxic AKI), thus improving the prognosis after the examination. That is to say, it is shown that the urinary MK concentration is useful as a marker for very early diagnosis of acute kidney injury (AKI).

### 6. Cases of Partial Nephrectomy for Superficial Early Kidney Cancer and Urinary Midkine (MK) Concentration

### (1) Subjects and Methods

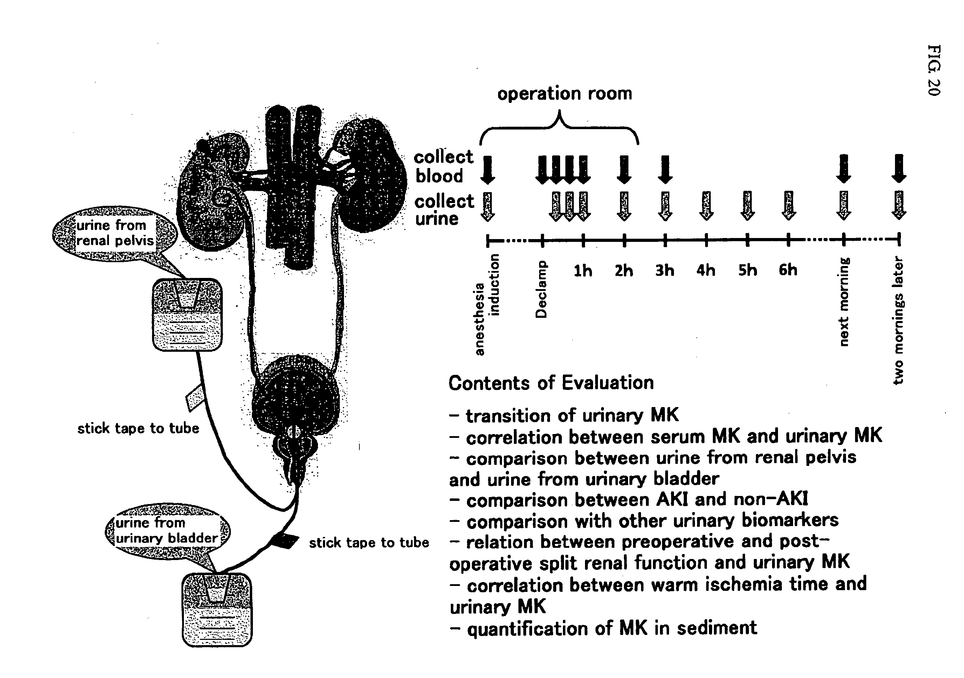

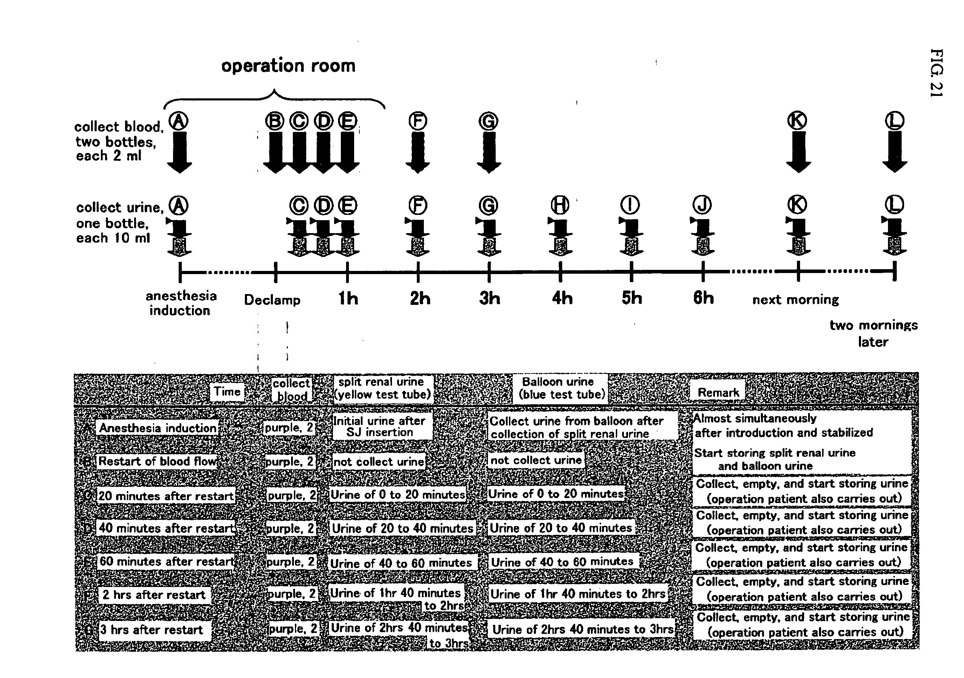

In partial nephrectomy for superficial early kidney cancer, during nephrectomy for kidney cancer portion, the affected renal artery was interrupted. After excision, renal blood flow was restarted. In this surgery, since the renal artery and the renal vein are completely blocked for about 30 minutes of partial nephrectomy of a tumor, ischemic kidney injury occurs in the affected kidney. Originally, the kidney has large reserve function, and even if total extirpation of one of the kidneys is carried out, generally, the other kidney compensates the extirpated kidney. Therefore, the Cr increase is limited. However, in some cases, kidney disorder due to this surgery is protracted. In this surgery, in order to carry out observation of postoperative bleeding or check of urine output, basic surgery procedure is carried out by inserting a catheter into the affected renal pelvis to collect the affected urine, and, on the other hand, collecting the urine derived from the healthy kidney from a catheter indwelling in the urinary bladder. According to the protocols shown in Figs. 20 and 21, preoperative and postoperative blood and urine were collected, and the urinary MK concentration was examined.

### (2) Results

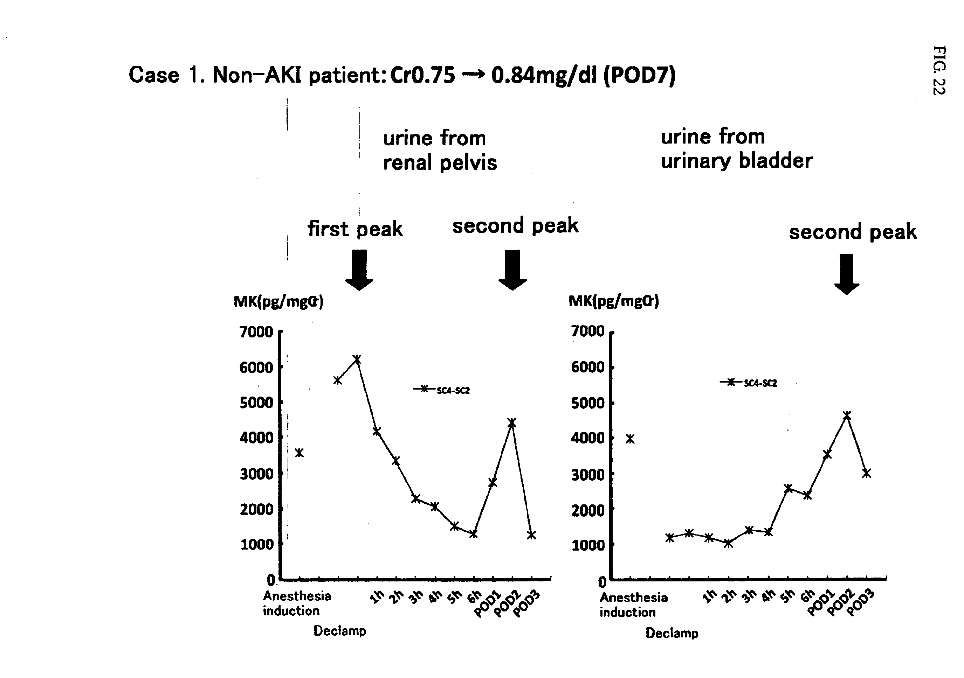

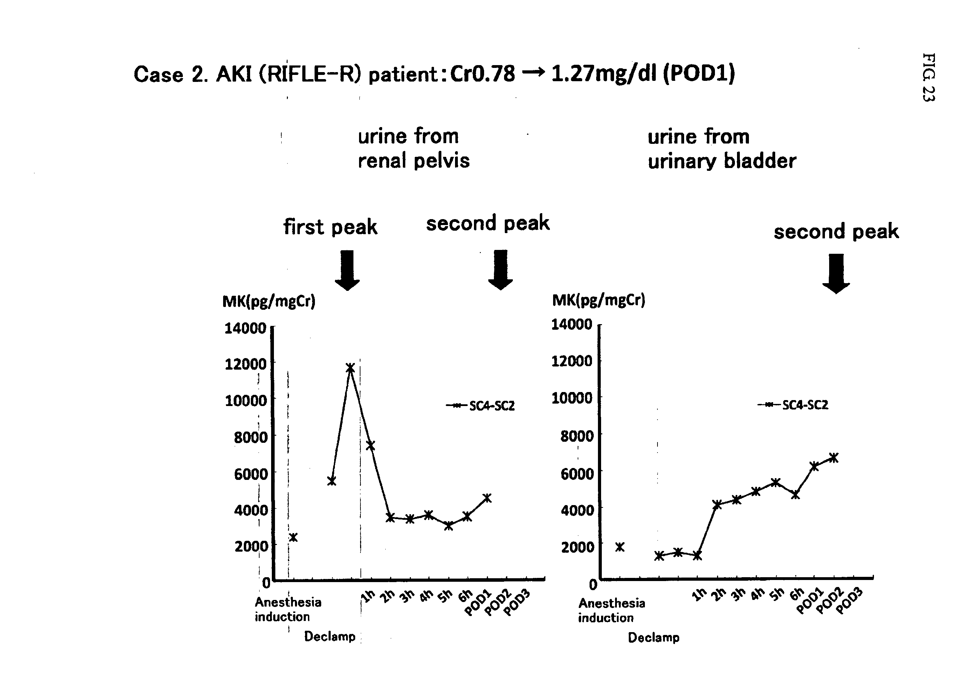

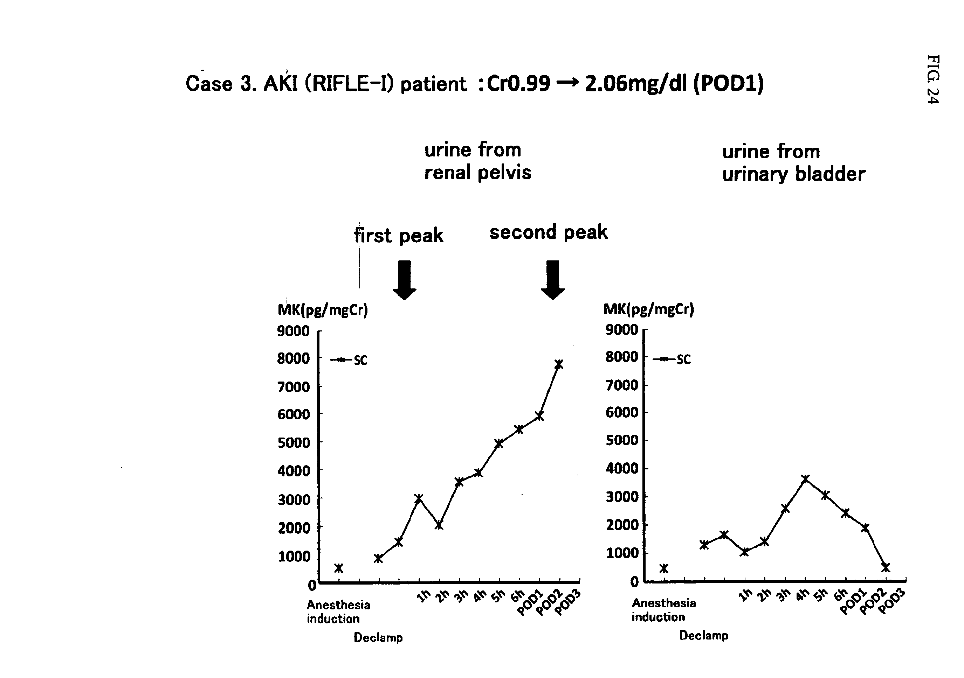

The urine derived from the excised kidney showed diphase peaks in the urinary MK concentration, that is, the first peak within one hour after the start of surgery and the second peak within 6 to 48 hours after the surgery. The second peak is also observed in the urine derived from the affected kidney and the healthy kidney. This thought to be because ischemic disorder occurring in the affected kidney extends to organs in the whole body including the healthy kidney, which reflect MK in which protein synthesis is newly promoted from the both kidneys. Therefore, in cases in which the renal function is slight, the first peak and the second peak show only transitional increase (Fig. 22). On the other hand, in elderly persons whose reserve function of the kidney is deteriorated or patients with chronic kidney diseases, renal dysfunction is protracted (Figs. 23 and 24). In this case, the second peak of the urinary MK concentration is not transitional, and the increased value is protracted and maintained. This is thought to be because production and secretion of MK protein are newly promoted by a transcriptional factor called HIF-1 responding to the stress by ischemia. This shows that in cases in which this second peak is protracted (cases 2 and 3), stress is maintained, that is, kidney disorder is strong. Thus, when the second peak is noted, estimation of prognosis is possible.

### (3) Discussion

As mentioned above, it is shown that the urinary MK concentration is useful as an early marker for acute kidney injury also in this disease group. Furthermore, it is proved that the urinary MK concentration is useful as a "marker for early diagnosis" but also "biomarker as a prediction factor of prognosis" for acute kidney injury.

### 7. Kidney Disorder Progressing after Chemotherapy or in Nephrotic Syndrome and Urinary Midkine (MK) Concentration

### (1)Subjects and Methods

The urinary MK concentration was measured in the cases in which renal dysfunction progressed and was protracted after chemotherapy using, for example, methotrexate (MTX) and during medical treatment of nephrotic syndrome.

### (2) Results

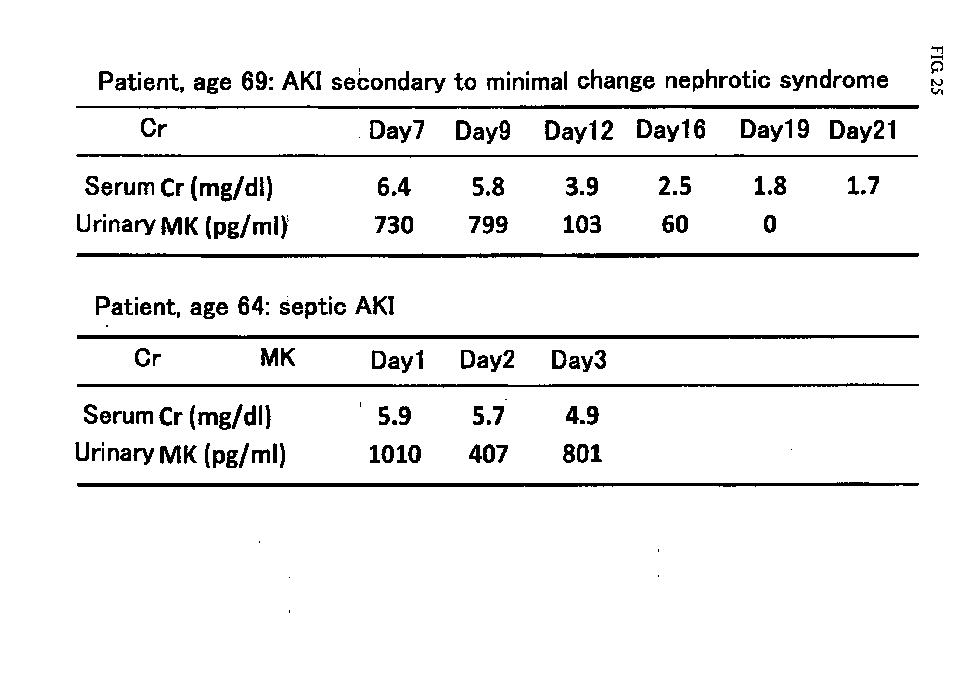

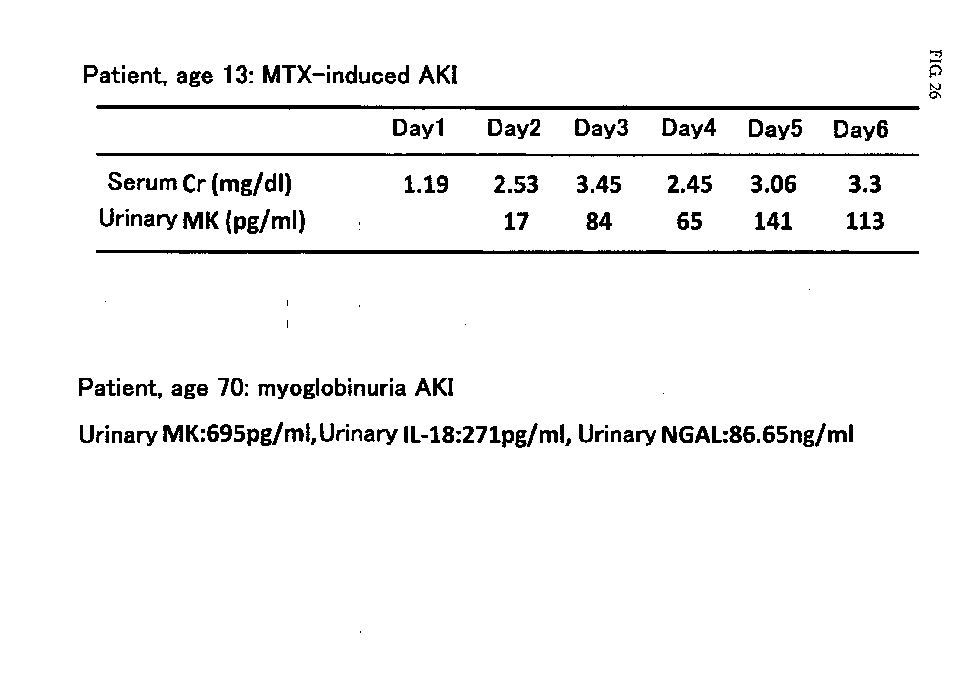

During the treatment of nephrotic syndrome, the urinary MK concentration maintained a high value (on day 7 to day 9 after kidney biopsy; urinary MK 730-770 pg/ml) in the term during which renal dysfunction was protracted. However, in accordance with the improvement of the renal function, the urinary MK concentration was reduced (upper table of Fig. 25). Also in patients with septic AKI, the same tendency is observed (lower table of Fig. 25). Furthermore, as shown in Fig. 26, also in the kidney disorder after chemotherapy using MTX, during the time in which renal dysfunction was maintained (on day 2 to day 6), the urinary MK concentration maintained a high value.

### (3) Discussion

In addition to the above-mentioned partial nephrectomy of renal cancer, in AKI that follows primary and secondary glomerular nephritis such as AKI and nephrotic syndrome after chemotherapy, it is shown that the urinary MK is useful as a "biomarker as a predicting factor of prognosis."

### 8. Minimal Change Nephrotic Syndrome and Urinary Midkine (MK) Concentration

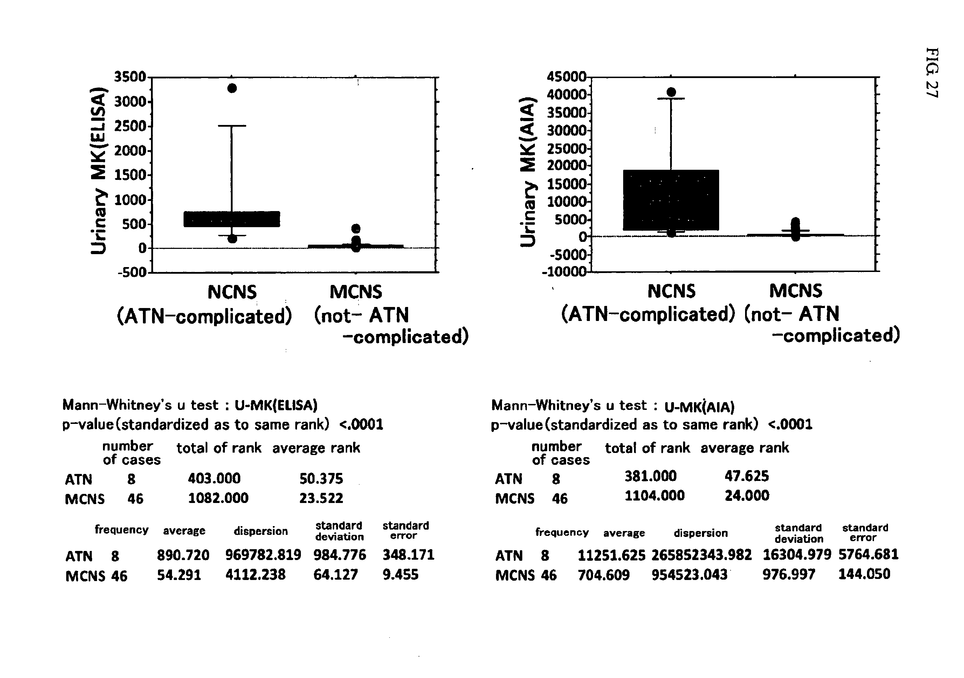

It is known that 10-20% of "minimal change" that is the most frequent cause presents acute tubular necrosis (ATN). The MK concentration of a urine sample collected at the time of kidney biopsy from the kidney with minimal change was examined. As a result, in acute tubular necrosis (ATN) complication cases, the MK concentration was significantly high (Fig. 27). Thus, it is shown that the urinary MK concentration is useful for early detection of cases progressing from the minimal change into ATN (AKI in a narrow sense).

### 9. Relation 1 between Urinary MK Concentration and Blood MK Concentration

### (1) Subjects and Methods

The urinary MK concentration and serum MK concentration were measured for 515 patients with nephritis (cross-section study). Furthermore, the urinary MK concentration and serum MK concentration were measured a plurality of times in 32 patients undergoing an abdominal aortic aneurysm replacement surgery (case control study). On the other hand, the affinity column of MK was prepared to attempt to identify the protein bonded to MK in the serum.

### (2) Results

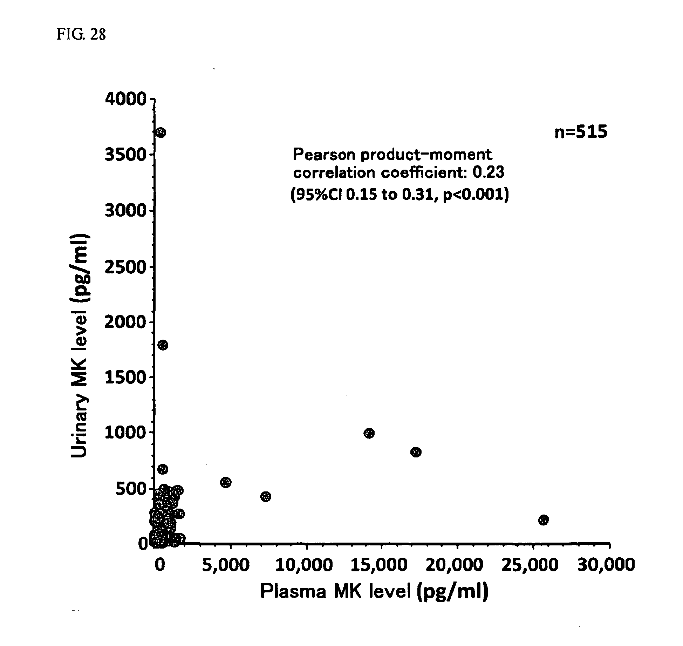

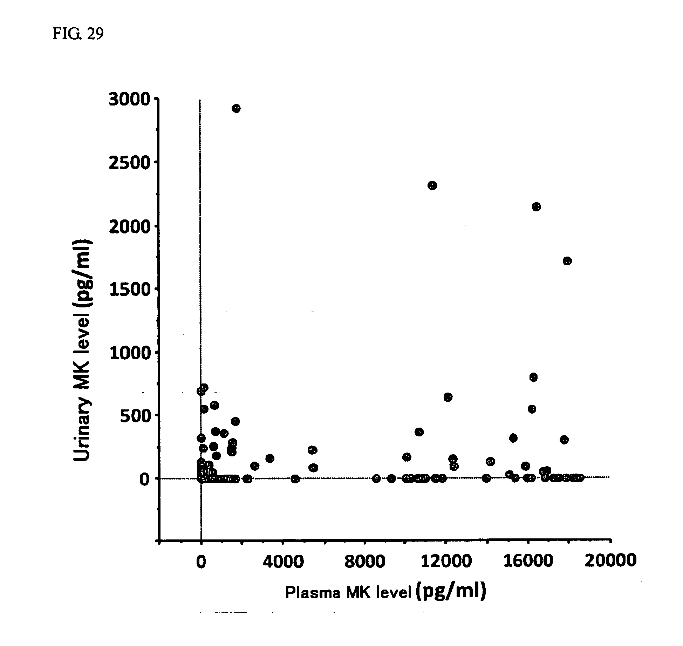

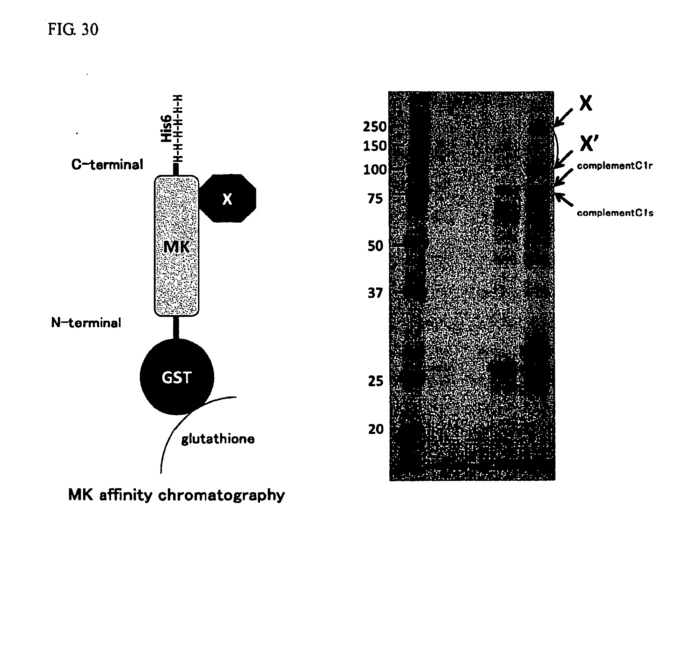

The measurement results of the urinary MK concentration and the blood MK concentration are shown in Figs. 28 and 29. It is shown that MK is not subjected to glomerular filtration. On the other hand, as a result of examination of components in serum bonded to affinity column of MK by electrophoresis (Fig. 30), it was predicted that high molecular weight protein having a molecular weight of about 250 kDa (band shown by X in the drawing and band shown by X' shown in the drawing was predicted to be a fragment of X) was bound to MK. For the protein, the molecule was identified by TOF-MAS. From these results, almost all of blood MK is bound to a heparin chain of the vascular endothelial cell, even if soluble MK is present, it binds to protein having a molecular weight of 250 kDa in blood. Therefore, it is thought that MK in blood it is not leaked into urine (since glomerular basement membrane has a size barrier (network pores of collagen fiber, radius is said to be about 70 angstrom), when the molecular weight is 250 kDa, it is not filtered).

### 10. Relation 2 of Urinary MK Concentration and Blood MK Concentration

### (1)Subjects and Methods

For patients with amyloid kidney-complicate chronic rheumatoid arthritis, comparison of the urinary MK concentration and the plasma MK concentration between with administration of heparin and without administration of heparin was carried out.

### (2) Results

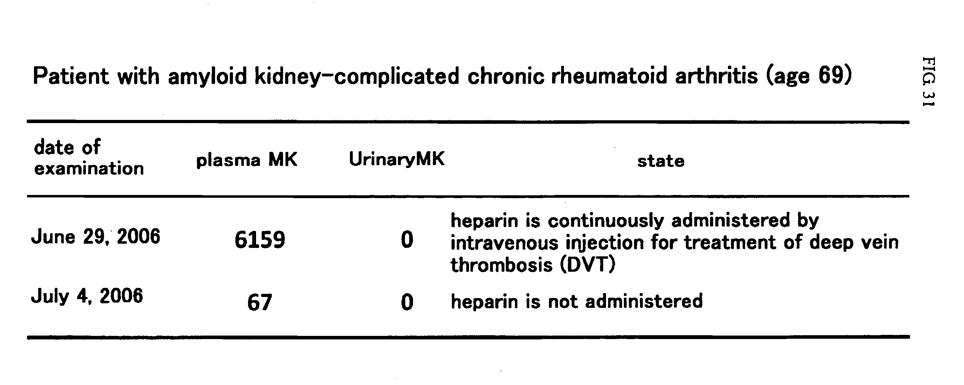

The MK concentration is surely increased by administration of heparin (Fig. 31), but MK is not detected in the urine even in a nephrosis state by amyloid kidney (glomerular basement membrane disorder is strong, and high molecule protein such as not only alb but also IgG leaks). That is to say, basically, it is thought that MK does not leak from the glomerulus.

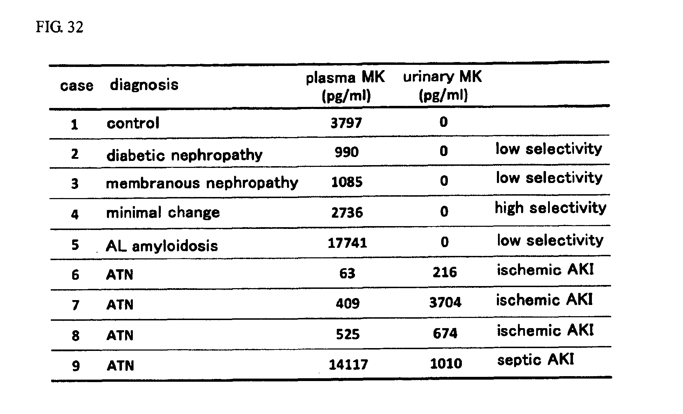

In cases 1 to 5 of Fig. 32, although the plasma MK concentration is high, MK is not detected in the urine. In particular, cases 2, 3, and 5 show disease in which the selectivity of protein is low (that is to say, a typical disease in which damage of the glomerular basement membrane is strong and permeation is promoted, thus making even a high molecule leak into the urine), which is worthy of attention. The cases 6 to 9 are ATN (AKI in a narrow sense) cases in which the urinary MK concentration is high, and the plasma MK concentration is various from low to high. Most of AKIs are basically developed from basic disease causing stress in the whole body (for example, septicemia or after surgery), and naturally, plasma MK may be increased in many cases. As mentioned above, it is supported that MK is not subjected to glomerular filtration.

### [Industrial Applicability]

In the examination method of the present invention, the possibility of the onset of AKI is determined by using an amount of urinary MK as an indicator. The examination method of the present invention permits very early prediction of the onset of AKI, which cannot be achieved by existing biomarkers (NGAL, IL-18, and the like). When the early prediction of AKI becomes possible, treatment can be intervened in an early stage, thus improving the prognosis. The urinary MK amount is also useful for estimation of prognosis associated with a renal function. By using the amount of urinary MK as an indicator, not only the possibility of the onset of AKI but also estimation of prognosis associated with a renal function can be carried out. The urinary MK has a different response from that of the existing biomarkers. By using the difference in the response, when the urinary MK and the existing biomarkers are used together, determination with higher accuracy and reliability can be carried out.

On the other hand, also in the differentiation method according to the disclosed exmplatory embodiment, by using the amount of urinary MK as an indicator, differentiation of AKI can be carried out. According to the differentiation method, differentiation of AKI can be carried out with extremely high sensitivity and specificity. By using both urinary MK and existing biomarker, accuracy and reliability can be improved.

## Claims

1. Use of a biomarker, comprising urinary midkine, in vitro for
early detection of acute kidney injury; wherein the acute kidney injury is acute tubular necrosis; and wherein urine is used as a specimen.

2. An in vitro method, wherein an amount of urinary midkine is used as an indicator, for
examining acute kidney injury;
wherein the acute kidney injury is acute tubular necrosis, and wherein urine is used as a specimen.

3. The method of examining acute kidney injury according to claim 2, comprising the following steps (1) and (2):
(1) detecting urinary midkine; and
(2) determining a possibility of the onset of acute kidney injury based on a detection result.

4. The method of examining acute kidney injury according to claim 2 or 3, wherein the acute kidney injury is acute tubular necrosis including ischemic acute kidney injury, toxic acute kidney injury, septic acute kidney injury or postoperative ischemic acute kidney injury.

5. The method of examining postoperative ischemic acute kidney injury according to claim 4, wherein the step (1) is carried out by using urine collected from a time of a surgery that causes an ischemic state to one day after the surgery; or by using urine collected in three hours from a start of the surgery that causes an ischemic state.

6. The method of examining postoperative ischemic acute kidney injury according to claim 4 or 5, wherein the surgery includes large blood vessel surgery, open heart surgery using an artificial heart lung machine, organ transplantation, or partial nephrectomy for a renal tumor.

7. The examination method according to any one of the claims 3 to 6, further comprising the following step (1') in addition to the step (1), wherein the step (2) determines the possibility of the onset of acute kidney injury based on the detection results of the step (1) and step (1'):
(1') detecting one or more biomarkers selected from the group consisting of urinary neutrophil gelatinase-associated lipocalin (NGAL), urinary interleukin 18 (IL-18), urinary kidney disorder molecule-1 (KIM-1), β-N-acetyl-D-glucosaminidase (NAG), and urinary liver type fatty acid-binding protein (L-FABP).

8. The examination method according to claim 3, wherein in the step (1) urinary midkine is detected by using urine of patients with minimal change.

9. Use of a reagent or a kit comprising said reagent for
examination of acute kidney injury;
wherein the acute kidney injury is acute tubular necrosis, comprising an anti-midkine antibody, and wherein urine is used as a specimen.

## Patentansprüche

1. Verwendung eines Biomarkers, umfassend Urin-Midkin, in vitro zur Früherkennung von akutem Nierenversagen; wobei das akute Nierenversagen akute tubuläre Nekrose ist; und wobei Urin als Probe verwendet wird.

2. In vitro Verfahren, wobei eine Menge von Urin-Midkin als Indikator verwendet wird, zur Untersuchung von akutem Nierenversagen;
wobei das akute Nierenversagen akute tubuläre Nekrose ist, und wobei Urin als Probe verwendet wird.

3. Verfahren zur Untersuchung von akutem Nierenversagen nach Anspruch 2, umfassend die folgenden Schritte (1) und (2):
(1) Nachweis von Urin-Midkin; und
(2) Bestimmung einer Möglichkeit des Ausbruchs eines akuten Nierenversagens basierend auf einem Ergebnis des Nachweises.

4. Verfahren zur Untersuchung von akutem Nierenversagen nach Anspruch 2 oder 3, wobei das akute Nierenversagen die akute tubuläre Nekrose ist, einschließlich ischämischen akuten Nierenversagens, toxisch bedingten akuten Nierenversagens, septischen akuten Nierenversagens oder postoperativen ischämischen akuten Nierenversagens.

5. Verfahren zur Untersuchung von postoperativen ischämischen akuten Nierenversagen nach Anspruch 4, wobei Schritt (1) durchgeführt wird durch Verwendung von Urin, welcher vom Zeitpunkt einer Operation, die einen ischämischen Zustand herbeiführt, bis zu einem Tag nach der Operation entnommen wurde; oder durch Verwendung von Urin, welcher drei Stunden vom Beginn der Operation, die einen ischämischen Zustand herbeiführt, entnommen wurde.

6. Verfahren zur Untersuchung von postoperativen ischämischen akuten Nierenversagen nach Anspruch 4 oder 5, wobei die Operation Operation an großen Blutgefäßen, Operation am offenen Herzen unter Verwendung einer künstlichen Herz-Lungen-Maschine, Organtransplantation oder partielle Nephrektomie eines Nierentumors einschließt.

7. Untersuchungsverfahren nach einem der Ansprüche 3 bis 6, ferner umfassend den folgenden Schritt (1') zusätzlich zu Schritt (1), wobei der Schritt (2) die Möglichkeit des Ausbruchs eines akuten Nierenversagens basierend auf den Ergebnissen des Nachweises des Schritts (1) und Schritts (1') bestimmt:
Urin-Interleukin-18 (IL-18), Urin-Kidney Injury Molecule-1 (KIM-1), β-N-Acetyl-D-Glucosaminidase (NAG) und Urin-Lebertyp Fettsäure-bindendem Protein (L-FABP).

8. Untersuchungsverfahren nach Anspruch 3, wobei im Schritt (1) Urin-Midkin durch Verwendung von Urin von Patienten mit minimalen Veränderungen nachgewiesen wird.

9. Verwendung eines Reagens oder eines Kits, umfassend das Reagens zur Untersuchung von akutem Nierenversagen;
wobei das akute Nierenversagen akute tubuläre Nekrose ist, umfassend einen Anti-Midkin Antikörper, und wobei Urin als Probe verwendet wird.

## Revendications

1. Utilisation d'un biomarqueur, comprenant la midkine urinaire, *in vitro* pour une détection précoce d'une lésion rénale aiguë ; où la lésion rénale aiguë est une nécrose tubulaire aiguë ; et où l'urine est utilisée comme échantillon.

2. Procédé *in vitro,* dans lequel une quantité de midkine urinaire est utilisé comme indicateur, pour l'examen d'une lésion rénale aiguë ;
où la lésion rénale aiguë est une nécrose tubulaire aiguë, et où l'urine est utilisée comme échantillon.

3. Procédé d'examen d'une lésion rénale aiguë selon la revendication 2, comprenant les étapes (1) et (2) suivantes :
(1) la détection de la midkine urinaire ; et
(2) la détermination de la possibilité de l'installation d'une lésion rénale aiguë basée sur un résultat de détection.

4. Procédé d'examen d'une lésion rénale aiguë selon la revendication 2 ou 3, dans lequel la lésion rénale aiguë est une nécrose tubulaire aiguë y compris une lésion rénale aiguë ischémique, une lésion rénale aiguë toxique, une lésion rénale aiguë septique ou une lésion rénale aiguë ischémique postopératoire.

5. Procédé d'examen d'une lésion rénale aiguë ischémique postopératoire selon la revendication 4, dans lequel l'étape (1) est réalisée en utilisant l'urine recueillie après un temps de chirurgie qui provoque un état ischémique jusqu'à un jour après la chirurgie ; ou en utilisant l'urine recueillie dans les trois heures qui suivent le début de la chirurgie qui provoque un état ischémique.

6. Procédé d'examen d'une lésion rénale aiguë ischémique postopératoire selon la revendication 4 ou 5, dans lequel la chirurgie comprend une chirurgie de gros vaisseau sanguin, une chirurgie à coeur ouvert utilisant une machine de coeur-poumon artificiel, une transplantation d'organe, ou une néphrectomie partielle pour une tumeur rénale.

7. Procédé d'examen selon l'une quelconque des revendications 3 à 6, comprenant en outre l'étape (1') suivante en plus de l'étape (1), dans lequel l'étape (2) détermine la possibilité de l'installation d'une lésion rénale aiguë basée sur les résultats de détection de l'étape (1) et de l'étape (1') :
(1') la détection d'un ou de plusieurs biomarqueurs choisis dans le groupe constitué par la lipocaline urinaire associée à la gélatinase des neutrophiles (NGAL), l'interleukine 18 (IL-18) urinaire, la molécule 1 urinaire des troubles rénaux (KIM-1), la β-N-acétyl-D-glucosaminidase (NAG), et la protéine urinaire de liaison des acides gras de type hépatique (L-FABP).

8. Procédé d'examen selon la revendication 3, dans lequel dans l'étape (1) la midkine urinaire est détectée en utilisant l'urine des patients avec un changement minime.

9. Utilisation d'un réactif ou d'un kit comprenant ledit réactif pour
l'examen d'une lésion rénale aiguë ;
où la lésion rénale aiguë est une nécrose tubulaire aiguë, comprenant un anticorps anti-midkine, et où l'urine est utilisée comme échantillon.


### SEQUENCE LISTING

<110> NATIONAL UNIVERSITY CORPORATION NAGOYA UNIVERSITY
<120> Novel Biomarker and uses thereof
<130> P08014P
<150> JP P2008-048954
   <151> 2008-02-29
<150> JP P2008-302443
   <151> 2008-11-27
<150> JP P2008-302444
   <151> 2008-11-27
<160> 1
<170> PatentIn version 3.5
<210> 1
   <211> 143
   <212> PRT
   <213> Homo sapiens
<400> 1
   Met Gln His Arg Gly Phe Leu Leu Leu Thr Leu Leu Ala Leu Leu Ala
   1               5                   10                  15
   Leu Thr Ser Ala Val Ala Lys Lys Lys Asp Lys Val Lys Lys Gly Gly
               20                  25                  30
   Pro Gly Ser Glu Cys Ala Glu Trp Ala Trp Gly Pro Cys Thr Pro Ser
           35                  40                  45
   Ser Lys Asp Cys Gly Val Gly Phe Arg Glu Gly Thr Cys Gly Ala Gln
       50                  55                  60
   Thr Gln Arg Ile Arg Cys Arg Val Pro Cys Asn Trp Lys Lys Glu Phe
   65                  70                  75                  80
   Gly Ala Asp Cys Lys Tyr Lys Phe Glu Asn Trp Gly Ala Cys Asp Gly
                   85                  90                  95
   Gly Thr Gly Thr Lys Val Arg Gln Gly Thr Leu Lys Lys Ala Arg Tyr
               100                 105                 110
   Asn Ala Gln Cys Gln Glu Thr Ile Arg Val Thr Lys Pro Cys Thr Pro
           115                 120                 125
   Lys Thr Lys Ala Lys Ala Lys Ala Lys Lys Gly Lys Gly Lys Asp
       130                 135                 140